# EUROPEAN PATENT APPLICATION

(11) **EP 1 481 670 A1**
(43) Date of publication of application: **01.12.2004**
(21) Application number: 02710424.9
(22) Date of filing: 31.01.2002
(51) Int. Cl.: A61K 31/122, A61K 31/341, A61P 3/04, A61P 3/06, A61P 3/10, A61P 9/12, C07D 307/32, C07D 307/58

(54) **MEDICINAL COMPOSITION FOR DIAGNOSIS, PREVENTION, OR THERAPY OF MULTIPLE RISK FACTOR SYNDROME**

(71) Applicant: aRigen, Inc., Minato-ku, Tokyo 107-0061 (JP)
(72) Inventor: ANDO, Kunio, c/o aRigen, Inc., Tokyo 107-0061 (JP); HOSOKAWA, Tomoyoshi, c/o aRigen, Inc., Tokyo 107-0061 (JP); YAMAMOTO, Masaichi, c/o aRigen, Inc., Tokyo 107-0061 (JP)
(74) Representative: Eddowes, Simon
(86) International application number: PCT/JP2002/000767
(87) International publication number: WO 2003/063849

(57) **Abstract**

A preventive or therapeutic agent for multiple risk factor syndrome containing, as an active ingredient, one or more chemical compounds selected from the group consisting of ascochlorin, ascochlorin homologues, ascofuranone and ascofuranone homologues,
each of the chemical compounds having at least an olsyl aldehyde moiety, in which
neither of the hydrogen atoms of the 2- and 4-hydroxyl groups are substituted; and
the hydrogen atom of at least one of the hydroxyl groups is substituted by a group selected from the group consisting of (C₁-C₁₅) alkyl, (C₂-C₁₅) alkenyl, (C₁-C₁₅) alkylnyl, aryl, (C₁-C₁₅) alkylaryl ; - (CₙH₂ₙ)R (where n is an integer of 1 to 15 and R represents COOR' which substitutes for any one of n carbon atoms where R' is H or (C₁-C₁₅) alkyl), (C₁-C₁₅) alkylcarbonyl, arylcarbonyl, aryl(C₁-C₁₅)alkyl, aryl (C₁-C₁₅) alkylcarbonyl, -C (=O) (CH₂)₁₋₁₅COOH, 2-pyridylcarbonyl, nicotinoyl, isonicotinoyl, toluenesulfonyl, carbamoyl, carbamoyl substituted by one or two (C₁-C₆) alkyls, amino, amino substituted by one or two (C₁-C₆) alkyls, aryloxy (C₁-C₁₅) alkyl optionally having a halogen on the ring, and aryl having a (C₁-C₆) alkoxy or a (C₁-C₆) alkoxy on the ring.

## Description

### TECHNICAL FIELD

The present invention relates to a pharmaceutical composition for diagnosis, prevention and therapy of multiple risk factor syndrome, containing ascochlorin, ascofuranone and derivatives thereof as active ingredients.

### BACKGROUND ART

### Multiple risk factor syndrome

### [Concept of multiple risk factor syndrome]

The multi risk factor syndrome is a relatively new concept of disease. This concept is defined based on the fact that risk factors such as hyperlipemia, obesity, essential hypertension, impaired glucose tolerance, hyperinsulinemia and the like (which have been found to cause diseases such as ischemic heart disease, type II diabetes, and cerebrovascular accident) are often integrated in a single individual. Behind the syndrome, insulin tolerance underlines which takes place in the peripheral tissues. Before starting explanation for the present invention, why such a new concept must be introduced, why research and development of a new medicament for treating such a syndrome has been desired, and so forth will be explained.

The leading cause of death in advanced countries only exclusive of Japan is heart disease. In most countries, heart disease occupies substantially 70% to 40% of death and thus overwhelms other causes of death, getting No.1 position. Accordingly, attention must be paid to the fact: among many diseases, heart disease occupies a completely different position in the Caucasian from the Japanese. In brief, for the Caucasian, heart disease is not only a primary matter of health concern but also a fear, which is even more than that of the Japanese who are afraid of cancer.

Most of the heart disease is ischemic heart disease caused when the arteria coronaria supplying nutrition and oxygen to the muscle of the heart is narrowed and clogged. In the arteria coronaria, a local inflammatory lesion takes place by various causes derived from lifestyle, aging, and hereditary nature. As a result, the blood vessel becomes narrow, decreasing blood flow to the heart muscle. In an extreme case, blood flow stops because of clogging. If so, ischemic heat diseases such as myocardial infarction and cardiac failure, frequently take place. Such an arterial lesion shows complicated pathological signs characterized by removal of blood-vessel inner membrane, cholesterol deposition on the blood-vessel tunica media, proliferation of the tunica media smooth muscle cells, and conversion of thrombus to the stroma. The lesion grows thick and gradually protrudes toward the center of the arterial lumen. Such a arterial lesion is called arteriosclerosis. In other than the arteria coronaria, arteriosclerosis possibly occurs in all types of arteries including the main artery, the carotid artery, the arteries of the kidney, the femoral region, and the lower extremities. However, generally, arteriosclerosis means arteriosclerosis of the arteria coronaria, which induces a serious heart disorder, and also called ischemic heart disease since blood supply to the cardiac muscle stops or reduces.

Most ischemic heart diseases are developed with the progress of arteriosclerosis. In the advanced countries of the Europe and the United States, cardiovascular diseases such as myocardial infarction and cardiac failure occupy the top cause of death. In the United States, ischemia heart disease is about 40% of all causes of death and has been at a top position since 1900 when accurate statistics were started. Accordingly, the number of patients is huge. In the United States, it is reported to reach about 60 million (the US Heart Association, 2001 version). Also in the advanced countries of Western Europe, ischemia heart disease occupies the leading cause of death. For example, in Berlin of 1970's, actually, 70% of dead people were due to ischemia heart disease. Only one exception of the advance countries is Japan. The onset rate (per unit population) of ischemia heart disease in Japan is 1/5 of the United State and 1/10 of Northern Europe. Therefore, Japan belongs to the area where occurrence of ischemia heart disease is the lowest in the world. However, also in Japan, the number of people died of a cardiovascular disease, that is, the number of people died of ischemia heart disease and cerebrovascular disease, exceeds that of cancer and thus occupies the top position (the annual report on Health, Labor and Welfare, version 13).

According to a simple correlation analysis of epidemiological data, the factors showing a strong correlation with the onset of ischemia heart disease are three: "hypercholesterolemia", "hypertension", and "smoking". In other words, three major risk factors for ischemia heart disease are hypertension, hypercholesterolemia, and smoking. In the United States, to avoid three major risk factors, educational activities were intensively performed, with the result that onset and death from ischemia heart disease significantly reduced. Nevertheless, recently the reduction becomes slow down. Then, reasons of the slow-down are now studied. To explain more specifically, if hypercholesterolemia and hypertension as well as smoking are treated, the onset and death from ischemia heart disease decrease to some level. However, the level is still high and never decreases. In the circumstances, it seems natural that researchers started reconsidering risk factors leading to ischemia heart disease.

With respect to the role of another possible risk factor, obesity, the view that obesity alone is not a risk factor strongly connected to ischemia heart disease has been dominated. However, in non-insulin dependent diabetes (NIDDM), it has been known that diabetes is quite frequently complicated by obesity, hypercholesterolemia, and hypertension, and that ischemia heart disease starts earlier than in non diabetic patients. Reaven (DIABATES 37:1595-1607, 1988) defined coronary arteriosclerosis accelerated by integration of these risk factors as "syndrome X". He reported that the risk of starting ischemic heart disease is high if the following six risk factors are integrated: (1) insulin tolerance, (2) impaired glucose tolerance, (3) hyperinsulinemia, (4) high VLDL triglyceridemia (neutral fat contained in very low density lipoprotein increases over the upper limit of a normal range), (5) low HDL cholesterolemia (cholesterol contained in high density lipoprotein decreases over the lower limit of a normal range) and (6) hypertension. On the other hand, Kaplan proposed "The Deadly Quartet theory" (Arch Intern. Med. 149: 1514-1520, 1989). He named it because if (1) upper-body obesity, (2) impaired glucose tolerance, (3) high triglyceridemia, and (4) hypertension are integrated in one and the same single individual, ischemia heart disease is frequently induced, leading to death. Behind these two theories, insulin tolerance may underlie in common.

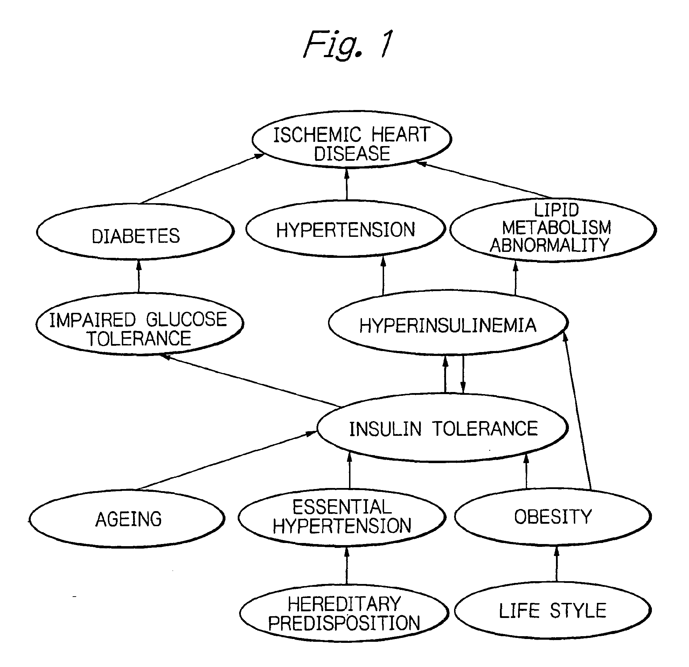

Then, DeFronzo combined both theories into one theory "insulin tolerance syndrome" in which he emphasizes that insulin tolerance is developed by obesity (Diabetes Care 14: 173-194, 1991). In Japan, Matsuzawa et al. investigated a risk factor of obesity in depth and proposed that if "visceral fat syndrome", caused by fat deposited onto the viscera, is complicated by hypertension, lipid metabolic error, and impaired glucose tolerance, the risk of developing ischemia heart disease increases (Journal of Japanese Society of Internal Medicine, 84: 1579-1582, 1995). Figure 1 shows the relationship of multiple risk factors. To explain more specifically, the factors underlining behind the multiple risk factor syndrome developing into ischemia heart disease are three: aging, hereditary predisposition, and life style. Of the three factors, aging and hereditary predisposition are factors uncontrolled at present. Only one controllable factor of them is life style such as smoking, overeating, shortage of exercise, and excessive intake of salt. Furthermore, the most serious problem resides in that a plurality of risk factors are likely to be integrated in one and the same individual, in other words, a patient having only one risk factor is rare. Taking obesity caused by overeating and shortage of exercise as an example, it has been so far considered that obesity itself is not a significant risk factor, but if it is integrated by impaired glucose tolerance, diabetes, lipid metabolic error, and hypertension, the risk of ischemic heart disease increases. However, recent studies on the systemic fat distribution revealed that upper-body obesity or a large amount of fat deposited on the viscera (the abdomen) induces hyperinsulinemia, and that impaired glucose tolerance, neutral-fat hyperlipemia, and hypertension are integrated with a high incidence. The risk of the fat deposited on the abdomen has been confirmed by a recent study using a transgenic mouse (Masuzawa H. et al., Science 294: 21662-2170, 2001). When glucocorticoid is produced excessively, causing fat deposition on the viscera, a large amount of free fatty acid is released from the fat, flows into the liver, and increases the synthesis of neutral fat and cholesterol. As a result, hyperlipemia is induced. The hyperlipemia exacerbates ischemia heart disease and diabetes. Obesity, hypertension, hyperlipemia, and impaired glucose tolerance are often integrated in one and the same individual. The rate of hypertension appearing in obese persons as a complication is three times as high as in non-obese persons. The rates of neutral-fat hyperlipemia and diabetes appearing in obese persons are twice as high as in non-obese persons, respectively.

Multiple risk factor syndrome proceeds without a subjective symptom. It is thus not a rare case that the syndrome is left alone without treatment. The syndrome is noticed all of a sudden when it reaches a life-threatening condition. Therefore, it is called a silent killer.

**Table 1.**

| Proponents for multiple risk factor syndrome and content | | | |
|---|---|---|---|
| Syndrome X (Reaven, 1988) | Visceral fat syndrome (Matsuzawa et al. 1987) | The deadly quartet (Kaplan, 1989) | Insulin tolerance syndrome (DeFronzo, 1991) |
| Insulin tolerance Hyperinsulinemia Impaired glucose tolerance Hypertriglyceridemia Low HDL cholesterolemia Hypertension | Impaired glucose tolerance Hypertriglyceridemia Low HDL cholesterolemia Hypertension Visceral fat accumulation | Imparted glucose tolerance Hypertriglyceridemia Hypertension Upper-body obesity | Hyperinsulinemia Non-insulin dependent diabetes Abnormal hyperlipemia Hypertension Obesity Arteriosclerosic heart disease |

### Insulin tolerance

It is pointed out that insulin tolerance underlies behind multiple risk factor syndrome. Insulin tolerance syndrome is often compared to an iceberg. Even if individual diseases of the insulin tolerance, such as diabetes and hypertension, protruding from the sea surface are mild, a serious danger may be hiding under the sea. Therefore, insulin tolerance syndrome cannot be fundamentally treated only by improving blood pressure and blood sugar alone. That is, needless to say, to treat multiple risk factor syndrome of a patient, insulin tolerance itself must be treated. Insulin is an only-one hormone capable of reducing a blood sugar level by converting glucose into energy. When insulin tolerance is induced by accumulation of fat in the viscera, the blood sugar level increases, developing diabetes. Furthermore, insulin tolerance invites "hyperinsulinemia" secreting a large amount of insulin. This is due to the function of the pancreas for compensating such a decreased effect of insulin by increasing the "amount". However, excessive amount of insulin causes the following phenomena: (1) it becomes difficult to excrete sodium ions from the kidney, with the result that water is stored within the body since it moves together with sodium ions; (2) fat is excessively bio-synthesized by the liver; and (3) the smooth muscle cells of the tunica media forming the blood-vessel wall proliferate and narrow the lumen of blood vessel. These phenomena each can directly lead to hypertension, hyperlipemia, and arteriosclerosis. A hyperinsulinemia patient becomes insulin tolerant more and more and such a vicious circle goes on, exacerbating a lifestyle-related illness.

### Animal model for multiple risk factor syndrome

As a model animal having multiple risk factors: 1. obesity, 2. high blood sugar, 3. hyperlipemia, 4. hypertension, and 5. hyperinsulinemia, there are known mouse models such as C57BL/6j-ob/ob(ob/ob mouse) and C57BL/ksj-db/db(db/db mouse) and a rat model such as Zucker fatty (Zucker rat). The ob/ob mouse was a mutation found in a C57BL/6J series black mouse by Ingalls et al., in 1950, whereas the db/db mouse was found in a C57BL/ksj black mouse by Hummel et al. in 1966 and established. Both mutations are transferred by way of single recessive genes, "ob" and "db". The Zucker rat is a mutation found by Zucker et al. in 1961 and established as being transferred by a recessive gene "fa". In the ob/ob mouse, a mutation of leptin expressed by an obesity gene and secreted from the adipose tissue is reported, whereas, in the db/db mouse as well as the Zucker fatty rat, an abnormality of a leptin receptor gene is reported. Commonly in the ob/ob mouse, db/db mouse, and Zucker fatty rat, obesity, an increase of white adipose cells in weight, hyperinsulinemia, high blood-sugar level, and neutral-fat hyperlipemia are observed.

The weight of db/db mice of 6 weeks old, is significantly high compared to normal Lean littermates and linearly increases with age up to 15 weeks old, and reaches 50 g, and thereafter starts decreasing. At 43 weeks old, there is no significant difference between the db/db mice and the Lean mice. The blood sugar level of the db/db mice of 6 weeks old is indicated twice as high as that of the Lean mice. Thereafter, the blood sugar level of the db/db mice continuously increases and reaches the peak as high as 700 mg/dL at 35 weeks old, the peak being maintained also thereafter. The blood insulin concentration of the db/db mice of 6 weeks old is several folds as high as the Lean mouse and reaches the peak at 15 weeks old, and thereafter rapidly decreases to a standard level of the Lean mice at 35 weeks old. The blood lipid level of the db/db mice is significantly high at any time, compared to the Lean mice. In the db/db mice, deaths probably due to circulatory failure are observed in younger generation than in the Lean mice. The average lifetime of db/db mice is shorter than the Lean mice.

The body weight and adipose tissue weight of 9-week old Zucker fatty rats is significantly higher than those of the Lean rats. They increase further thereafter virtually linearly. At 52 weeks old, the body weight of the Zucker rats reaches 700 g and the adipose tissue weight reaches 4 folds as high as that of the Lean rats. The non-fasting blood sugar level of the Zucker fatty rats tends to be higher than that of the Lean rats, however, there is no significant difference between them, whereas, the fasting blood sugar level of the Zucker rats is significantly higher than the Lean rat. In short, impaired glucose tolerance is observed. The blood insulin concentration of 9-week old mice is already three folds as high as that of the Lean rats and linearly increases until 24 weeks old, and thereafter, gradually decreases. The blood cholesterol, neutral fat, and free fatty acid are significantly higher in Zucker rats of 9 weeks old, than the Lean rats and linearly increase up to 52 weeks old. In particular, the fatty rats exhibit a remarkable symptom of neutral-fat hyperlipemia. The blood pressure of the fatty rats of 25 to 28 week olds is 20 to 30 mmHg which is higher than the Lean rats. The high blood pressure state is maintained further thereafter.

In the Lean rats, no histopathological change is observed in the aorta when cholesterol-rich feed is given, whereas, in the fatty rats, the cholesterol content in the aorta significantly increases and foam cells are observed.

### Epidemiology of multiple risk factor syndrome

Many reports have been made on risk factors for circulatory diseases. Some reports made it known that diseases such as hyperlipemia, hypertension, diabetes, and obesity are integrated in one and the same patient, and elucidated that insulin tolerance underlies behind these diseases. Different names are given to such morbidity depending upon proponents, for example, as Syndrome X, the Deadly Quartet, visceral fat syndrome, and multiple risk factor syndrome. Conventional analyses for risk factors are not performed systematically. No consideration is given to a time-dependent factor, which will bring critical information for elucidating onset-causes of circulatory diseases. In these circumstances, a working group for studying and finding a measure for overcoming work-related disease was organized under a commission from the Ministry of Health and Welfare. The working group analyzed health-check results of 122,051 workers and picked up 193 cases where sudden death and circulatory disorder were recorded in three years before January of 1990. The cases were analyzed by going back 10 years. Of risk factors excluding high cholesterol, such as obesity, blood pressure, blood sugar, and serum neutral fat level, how many abnormal factors are integrated in one and the same person was analyzed. This was compared to data of a risk-free individual to computationally obtain the risk of developing a circulatory disease. The results revealed that a multiple risk factor group having 3-4 of 4 risk factors integrated per person has a significantly high risk, which is as high as 30 times or more.

To summarize, it has been known that if hypertension, diabetes, neutral-fat hyperlipemia, and so forth are integrated and complicated with each other, even though each symptom is mild, a circulatory disease takes place at a high rate. More specifically, it has been elucidated that multiple risk factor syndrome having a plurality of risk factors (such as hypertension, diabetes, obesity, and neutral fat hyperlipemia) integrated therein is a major cause of a circulatory disease.

### Conventional therapy

Medicine is a foreign matter to a living body. No matter excellent medicine is developed as a result of progression of medical science and pharmacology, there is no good medicine over exercise therapy and dietary therapy. Therefore, in treating multiple risk factor syndrome, exercise therapy and dietary therapy is first chosen. Medicament is an option in the case where excise therapy and dietary therapy produce no effect or little effect. However, to treat a patient of multiple risk factor syndrome, it is not desirable to apply symptomatic treatment, in other words, to give a medicament to the patient in accordance with each of the symptoms in any cases. This is because simultaneous administration of medicaments, (for example, an antihypertensive drug to hypertension, a fibrate series medicament to hypertriglyceridemia, a statin series medicament to hypertension, a glytazone series medicament to overcome insulin tolerance, a central anorexigenic agent for reducing appetite and an absorption inhibitor for inhibiting ingested nutrition from being absorbed from the gastrointestinal tract, for treating obesity, and an aldosterone antagonist for treating edema), causes complicated interaction between the medicaments, with the result that efficacies of individual medicaments are inhibited from each other and, in an extreme case, it is even possible that toxicity of a medicament is augmented. On the other hand, insulin tolerance is located in the center of the multiple risk factor syndrome. Needless to say, priority must be given to overcoming insulin tolerance in order to treat the syndrome.

### Exercise therapy and dietary therapy

Many reports suggest that exercise therapy significant improves insulin tolerance. Exercise includes aerobics and anaerobics, both of which are effective. Of them, aerobics, specifically, jogging and power walking, seems to improve insulin tolerance more effectively. Exercise therapy is performed, in principle, for 20 minutes per time while maintaining 50% of maximum oxygen intake (VO₂max) (in a case of 30 to 40 year-old person, exercise must be performed so as to maintain a pulse of 120 to 130 per minute), and repeated three times per week. However, exercise therapy is not allowed to apply to some of the patients having ischemic heart disease, diabetes, and hypertension. Exercise therapy is therefore applied not all of the patients. As to dietary therapy, the therapy for insulin tolerance is basically planned so as not to accumulate body fat. That is, low-calorie and low-fat diet is given; however, crash diet is sometime countereffective, since rebound may occur afterward. Dietary habit is a life style primarily too difficult to change, so that it is difficult to apply a low calorie dietary therapy for a long time. In consideration of therapeutic effect, it is impossible to expect much on dietary therapy.

### Appetite suppressant and digestion/absorption inhibitor

Medicinal therapies for improving insulin tolerance are classified into indirect drug therapy and direct medicinal therapy. Examples of a medicament for indirectly improving insulin tolerance include an apatite suppressant and a digestion/absorption inhibitor. In our country, as a medicament for treating morbid obesity, a central appetite suppressant, mazindol, is limitedly applied to severe obesity having a body mass index (BMI) over 35%. However, because of a strong side effect, the application of the drug is permitted only within three months. Therefore, this medicament is virtually of no help to treat morbid obesity. On the other hand, as a diabetes therapeutic medicament, α-glucosidase inhibitors (Bogribose, Acarbose) are approved. The inhibitors are said to be able to suppressing the absorption of carbohydrates through the intestinal tract improve hyperinsulinemia after meals, thus becomes effective as indirect treatments for insulin tolerance when glucotoxicity is reduced. These inhibitors are medicaments which inhibit carbohydrate digestive enzymes, thereby suppressing absorption from the small intestine. In Europe and the United states where dietary energy is dependent mostly upon neutral fat of food, such a carbohydrate absorption inhibitor has little effect on overcoming insulin tolerance. As a medicament for inhibiting absorption of neutral fat from the gastrointestinal tract, an inhibitor for the activity of pancreas lipase, namely, Orlistat, is used. Orlistat inhibits neutral fat from being hydrolyzed into a fatty acid and a monoglyceride by pancreas lipase. It is said that 30% of dietary fat is not absorbed and excreted. In either case, these gastrointestinal tract adsorption inhibitors prevent nutrients such as carbohydrate and lipid from being digested and absorbed from the small intestine and allow a large amount of such undigested and absorbed nutrients to flow into the large intestine, thereby causing "microbial substitution of intestinal bacteria and abnormal proliferation of bad bacteria", "generation of a large amount of gas", and " abdominal distention/diarrhea". Because of such side effects, these inhibitors are limited in their use.

### Biguanide series medicament

A biguanide agent represented by Metformin and Buformin suppresses glycogenesis and glycogenolysis of the liver and increasing glucose uptake in the muscle, thereby improving insulin tolerance. The fact that phenformin belonging to this series of agent induces lethal lactate acidosis have been continuously reported in 1970's, and thus, use of a biguanide agent significantly reduced at a time. However, a large-scale diabetes study (UKPDS) has recently made and reported that this agent is effective and safe. Since then, this agent has come back as a medicament for improving insulin tolerance and put in wide use. However, since the liver is a major working site, a biguanide agent has the following big problems: liver failure takes place, lactate acidosis takes place, and the poisonous amount and the effective amount are indistinguishable.

### Thiazolidine series medicament

A thiazolidine series medicament, troglitazone, first came on the market in 1997 as a medicament for directly improving insulin tolerance. This agent has not only an effect of improving insulin sensitivity based on a different action from a conventional peroral diabetes medicament to reduce blood pressure, but also versatile effects. Based on this, it was considered that this agent may effectively work for treating insulin tolerance syndrome. However, side effects such as server liver failure were continuously reported and in addition, death cases were reported, so that use of this agent significantly decreased. Finally, discontinuation of sales of this agent was decided on March 22, 2000 and all products were withdrawn from the market. In a clinical field, the same thiazolidine series medicaments as troglitazone, namely, pioglitazone and rosiglitazone, were put on the market in 1999, and expected as an insulin tolerance therapeutic medicament. However, there medicaments have a possibility of inducing severe liver failure more or less and periodical observation of hepatic function is indispensable.

On the other hand, a thiazolidine series agent is reported to overcome insulin tolerance of the peripheral tissue. Nevertheless, three contradicting phenomena to the fact that insulin tolerance is overcome by this agent are reported by clinical statistic. The first phenomenon is that the blood pressure of type II diabetes is not lowered even if a thiazolidine series medicament is administered. On the contrary, many reports point out that blood pressure is increased because excretion of sodium ions from the kidney is inhibited, increasing amount of body fluid. Hypertension observed in about 70% of type II diabetes patients should be derived from insulin tolerance. Therefore, if insulin sensitivity can be systemically recovered, blood pressure should decrease as a natural consequence. This phenomenon is interpreted that a thiazolidine series medicament is not always overcome systemic insulin tolerance. The second phenomenon is that, as is clearly described in "warning on side effect", when a thiazolidine series medicament is administered, edema develops. The edema is considered to develop because the amount of circulatory blood plasma increases. It sometimes happens that heart failure gets worse or newly occurs, so that administration of this agent must be performed under sufficient observation. If edema, an abrupt increase of body weight and heart failure are observed, administration must be stopped. The frequency of occurrence of edema is 9.3% (33/353 cases) when accompanied with diabetic retinopathy requiring intensive care for overcoming insulin tolerance, 10.5% (29/276 cases) when accompanied with diabetic neuropathy, and 10.0% (23/230 cases) when accompanied with diabetic nephropathy, as well as tends to be high in diabetes with a complication than that without a complication. Since the safety of a thiazolidine medicament is in question, it is difficult to apply such a thiazolidine medicament to a diabetes patient having a complication. The third phenomenon is that administration of a thiazolidine series medicament increases body weight. In other words, fat deposition on the upper body or the internal organ increases against expectation. Since the dietary therapy for Type II diabetes is directed to reducing body weight, the weight increasing effect is a side effect against therapy.

### Antihypertensive agent

Examples of the antihypertensive agent to be administered to multiple risk factor syndrome include an angiotensin-converting enzyme inhibitor (ACE inhibitor), an angiotensine II-antagonist, and a calcium antagonist. As long as these agents are used exclusively in hypertensive patients having no complication, their safety is so high that they are used for a long time. As a matter of fact, however, a hypertensive patient simply having a problem of high blood pressure is rarely found and most hypertensive patients have several complications, that is, they are patients of multiple risk factor syndrome. Therefore, administration of an antihypertensive agent is effective in decreasing blood pressure but ineffective in preventing death from a cerebrovascular disease and ischemia heart disease, as compared before. This is because, in consideration of the working mechanism of an antihypertensive agent, an effect of recovering insulin sensitivity cannot be expected.

At present, there is no single medicament that overcomes insulin tolerance, thereby improving multiple risk factors such as hypertension, lipid metabolism error, impaired glucose tolerance, and obesity.

### DISCLOSURE OF THE INVENTION

As described above, multiple risk factor syndrome cannot be treated with a single medicament at present. However, provided that insulin tolerance is underlying behind the syndrome, a medicament that improves insulin tolerance should improve "hypertension", "lipid metabolic. error", "impaired glucose tolerance", "hyperinsulinemia" and "obesity" and further ischemia heart disease in which these risk factors appear in integrated form. The inventors of this patent have long studied ascochlorin, ascofuranone and derivatives thereof (hereinafter, referred to as a "compound of this series") with respect to the effect of improving insulin tolerance through animal experiment and clinical investigation. They found that these compounds overcome insulin tolerance, thereby significantly improving the morbidity of multiple risk factor syndrome. Based on the finding, they have accomplished the present invention.

The chemical compounds included in the present invention are ascochlorin described in Japanese Patent Application Nos. 10-377905 and 11-106996; and homologues thereof; or ascofuranone and homologues thereof.

The present invention provides a preventive or therapeutic agent for multiple risk factor syndrome; a process for preventing or treating multiple risk factor syndrome; a kit for preventing or treating multiple risk factor syndrome; use of the compounds for manufacturing a preventive or therapeutic agent for multiple risk factor syndrome; and a pharmaceutical composition containing a novel ascofuranone derivative, a novel ascochlorin derivative, one or more derivatives thereof; and a preventive or therapeutic agent for multiple risk factor syndrome, containing one or more of these derivatives as an active ingredient, described in items 1 to 10 below.
1. A preventive or therapeutic agent for multiple risk factor syndrome containing, as an active ingredient, one or more chemical compounds selected from the group consisting of ascochlorin, ascochlorin homologues, ascofuranone and ascofuranone homologues,
   each of the chemical compounds having at least an olsyl aldehyde moiety, in which
   neither of the hydrogen atoms of the 2- and 4-hydroxyl groups are substituted; and
   the hydrogen atom of at least one of the hydroxyl groups is substituted by a group selected from the group consisting of (C₁-C₁₅) alkyl, (C₂-C₁₅) alkenyl, aryl, (C₁-C₁₅) alkylaryl, -(CₙH₂ₙ)R (where n is an integer of 1 to 15, and R represents COOR' which substitutes for any one of n carbon atoms where R' is H or (C₁-C₁₅ ) alkyl), (C₁-C₁₅) alkylcarbonyl, arylcarbonyl, aryl(C₁-C₁₅)alkyl, aryl (C₁-C₁₅) alkylcarbonyl, -C(=O)(CH₂)₁₋₁₅COOH, 2-pyridylcarbonyl, nicotinoyl, isonicotinoyl, toluenesulfonyl, carbamoyl, carbamoyl substituted by one or two (C₁-C₆) alkyls, amino, amino substituted by one or two (C₁-C₆) alkyls, aryloxy (C₁-C₁₅) alkyl optionally having a halogen on the ring, and aryl having a (C₁-C₆) alkoxy or a (C₁-C₆) alkoxycarbonyl on the ring.
2. The preventive or therapeutic agent for multiple risk factor syndrome according to item 1, in which the compound is represented by the formula: 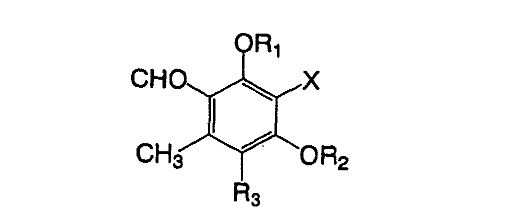 where X is represented by 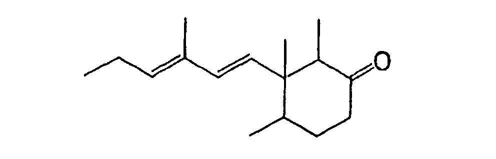 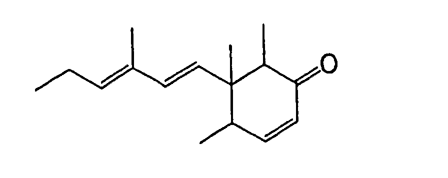 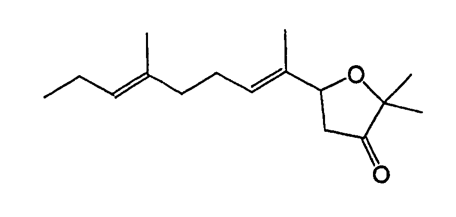 , or 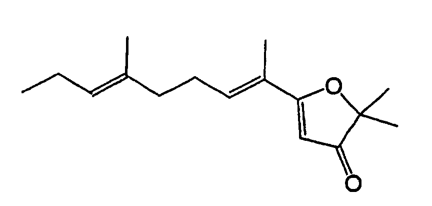 ;
   R₁ is H, (C₁-C₁₅) alkyl, (C₂-C₁₅) alkenyl, or (C₁-C₁₅) alkycarbonyl;
   R₂ is H, (C₁-C₁₅) alkyl, (C₂-C₁₅) alkenyl, aryl, (C₁-C₁₅) alkylaryl, -(CₙH₂ₙ)R (where n is an integer of 1 to 15 and R represents COOR' which substitutes for any one of n carbon atoms where R' is H or (C₁-C₁₅) alkyl), (C₁-C₁₅) alkylcarbonyl, arylcarbonyl, aryl (C₁-C₁₅)alkyl, aryl (C₁-C₁₅) alkylcarbonyl, -C(=O)(CH₂)₁₋₁₅COOH, 2-pyridylcarbonyl, nicotinoyl, isonicotinoyl, toluenesulfonyl, carbamoyl, carbamoyl substituted by one or two (C₁-C₆) alkyls, amino, amino substituted by one or two (C₁-C₆) alkyls, aryloxy (C₁-C₁₅) alkyl optionally having a halogen on the ring, and aryl having a (C₁-C₆) alkoxy or a (C₁-C₆) alkoxy on the ring; and
   R₃ is H or a halogen,
   with the proviso that a case where X is represented by the formula: 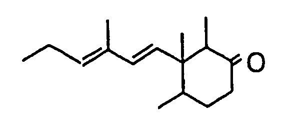 R₁ is H, R₂ is H, and R₃ is Cl is excluded.
3. The preventive or therapeutic agent for multiple risk factor syndrome, according to item 2, in which the compound is represented by the formula: 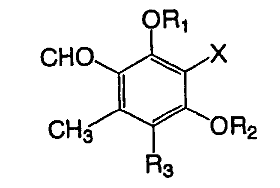 where X is represented by 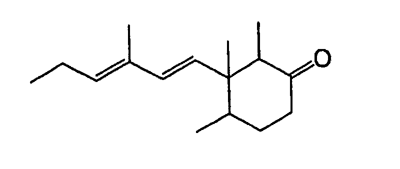 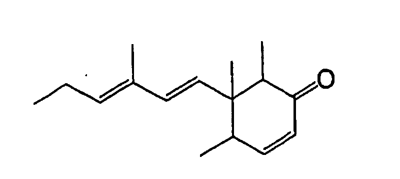 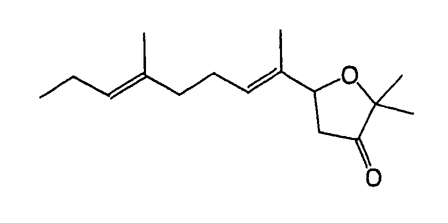 or 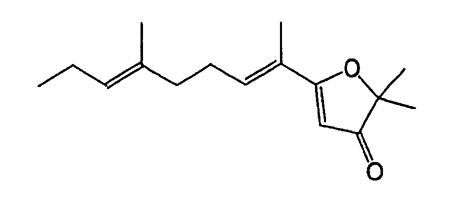 ;
   R₁ is H, (C₁-C₆) alkyl, (C₂-C₆) alkenyl, or (C₁-C₆) alkycarbonyl;
   R₂ is H, (C₁-C₆) alkyl, (C₂-C₆) alkenyl, aryl, (C₁-C₆) alkylaryl, (CₙH₂ₙ) R (where n is an integer of 1 to 6, and R represents COOR' which substitutes for any one of n carbon atoms where R' is H or (C₁-C₆) alkyl), (C₁-C₆) alkylcarbonyl, arylcarbonyl, aryl (C₁-C₆) alkyl, aryl(C₁-C₆) alkylcarbonyl, -C(=O)(CH₂)₁₋₆COOH, 2-pyridylcarbonyl, nicotinoyl, isonicotinoyl, toluenesulfonyl, carbamoyl, carbamoyl substituted by one or two (C₁-C₃) alkyls, amino, amino substituted by one or two (C₁-C₃) alkyls, aryloxy (C₁-C₆) alkyl optionally having a halogen on the ring, and aryl having a (C₁-C₃) alkoxy or a (C₁-C₃) alkoxy on the ring; and
   R₃ is H or Cl,
   with the proviso that a case where X is represented by the formula: 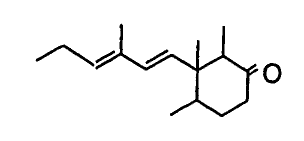 ,
   R₁ is H, R₂ is H, and R₃ is Cl, is excluded.
4. A preventive or therapeutic method for multiple risk factor syndrome comprising administering one or more chemical compounds according to any one of items 1 to 3 to a patient requiring therapy.
5. A kit for a preventive or therapeutic agent for multiple risk factor syndrome containing one or more chemical compounds according to any one of items 1 to 3 and an instruction for use.
6. Use of a compound according to any one of items 1 to 3 for manufacturing a preventive or therapeutic agent for multiple risk factor syndrome.
7. A compound represented by the following formula, a stereoisomer or a pharmaceutically acceptable salt thereof: 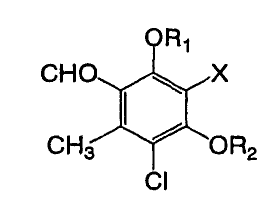 ;
   where X is 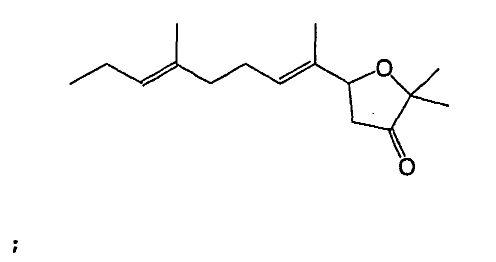 R₁ is H, (C₁-C₆) alkyl, (C₂-C₆) alkenyl, or (C₁-C₆) alkycarbonyl;
   R₂ is H, -CH₂COOH, -CH₂COO(C₁-C₅)alkyl, (C₁-C₆) alkycarbonyl, -C(=O)(CH₂)₁₋₆COOH, 2-pyridylcarbonyl, nicotinoyl, or isonicotinoyl; and
   R₃ is H or Cl,
   with the proviso that a case where R₁ is H, R₂ is H, and R₃ is Cl, is excluded; or
   X is represented by the formula: 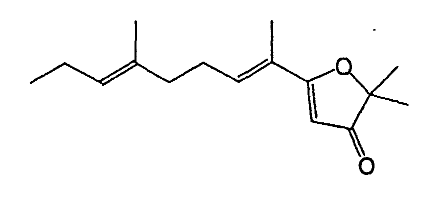 ,
   R₁ is H, (C₁-C₆ ) alkyl, (C₂-C₆) alkenyl, or (C₁-C₆) alkycarbonyl;
   R₂ is H, (C₁-C₆) alkyl, (C₂-C₆) alkenyl, -CH₂COOH, -CH₂COO(C₁-C₆)alkyl, (C₁-C₆) alkycarbonyl, -C(=O)(CH₂)₁₋₆COOH, 2-pyridylcarbonyl, nicotinoyl, or isonicotinoyl; and
   R₃ is H or Cl.
8. The compound represented by the following formula, a stereoisomer or a pharmaceutically acceptable salt thereof: 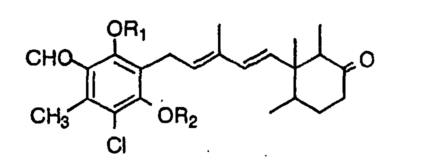 where R₁ is H, - (C₁-C₅) alkyl or alkenyl, or -C(=O-)(C₁-C₅)alkyl; and
   R₂ is H, -(C₁-C₅) alkyl, -CH₂COOH, -CH₂COO(C₁-C₅)alkyl, -C(=O)(C₁-C₅)alkyl, -C(=O)(CH₂)₁₋₅COOH, nicotinoyl, or isonicotinoyl,
   with the proviso that a case where R₁ is H, R₂ is -CH₂COOH, or nicotinoyl is excluded.
9. A pharmaceutical composition containing one or more chemical compounds according to item 7 or 8 and a pharmaceutically acceptable carrier.
10. A preventive or therapeutic agent for multiple risk factor syndrome, containing one or more chemical compounds according to item 7 or 8, as an active ingredient.

### BRIEF DESCRIPTION OF THE DRAWINGS

Figure 1 is an illustration showing the relationship of multiple risk factors inducing ischemic heart disease;
Figure 2 is a graph showing a time-dependent change of blood sugar level in a glucose tolerance test; and
Figure 3 is a graph showing a time-dependent change of urine sugar level in glucose tolerance test.

### BEST MODE FOR CARRYING OUT THE INVENTION

A chemical compound used as a preventive or therapeutic agent for multiple risk factor syndrome of the present invention and selected from the group consisting of ascochlorin, ascochlorin homologues, ascofuranone and ascofuranone homologues has at least one olsyl aldehyde moiety, in which
neither of the hydrogen atoms of the 2- and 4-hydroxyl groups are substituted;
the hydrogen atom of at least one of the hydroxyl groups is substituted by a group selected from the group consisting of (C₁-C₁₅) alkyl, (C₂-C₁₅) alkenyl, (C₁-C₁₅) alkynyl, aryl, (C₁-C₁₅) alkylaryl, -(CₙH₂ₙ)R (where n is an integer of 1 to 15 and R represents COOR' which substitutes for any one of n carbon atoms where R' is H or (C₁-C₁₅) alkyl), (C₁-C₁₅) alkylcarbonyl, arylcarbonyl, aryl (C₁-C₁₅)alkyl, aryl (C₁-C₁₅) alkylcarbonyl, -C(=O)(CH₂)₁₋₁₅COOH, 2-pyridylcarbonyl, nicotinoyl, isonicotinoyl, toluenesulfonyl, carbamoyl, carbamoyl substituted by one or two (C₁-C₆) alkyls, amino, amino substituted by one or two (C₁-C₆) alkyls, aryloxy (C₁-C₁₅) alkyl optionally having a halogen on the ring, and aryl having a (C₁-C₆) alkoxy or a (C₁-C₆) alkoxy on the ring.

In the present invention, the (C₁-C₁₅) alkyl represents a linear or branched alkyl group having 1 to 15 carbon atoms, preferably 1 to 8, more preferably 1 to 6. Preferable examples include methyl, ethyl, n-propyl, I-propyl, n-butyl, s-butyl, i-butyl, t-butyl, n-pentyl, n-hexyl, n-heptyl, n-octyl, n-nonyl, n-decyl, n-undecyl, and n-dodecyl. These groups may have substituents including halogens such as chloro(Cl), bromo (Br), and iode (I); hydroxyl (OH), and amino (NH₂).

In the present invention, the (C₁-C₁₅)alkenyl represents a linear or branched alkenyl group having a single double bond and 2 to 15 carbon atoms, preferably 2 to 8, more preferably 2 to 6. Preferable examples include vinyl, allyl, 1- or 2-butenyl, 1- or 2-pentenyl, 1-, 2-, or 3-hexenyl, and 1-, 2-, 3-, or 4-octenyl. Note that there groups may have substituents including halogens such as chloro (Cl), bromo (Br), and iode (I); hydroxyl (OH), and amino (NH₂).

In the present invention, the aryl represents an atomic group of aromatic compound from which a single hydrogen atom has been removed and refers to phenyl, naphthyl, biphenyl, anthranyl (anthryl), phenanthryl, and these groups substituted by 1 to 3 substituents (except alkyl). Although the types of the substituents are not particularly restricted, preferable substituents include (C₂-C₆) alkenyl, halogens such as fluoro, chloro, bromo, or iode, hydroxyl, amino, and nitro. Preferable aryl are phenyl and naphthyl.

In the present invention, the (C₁-C₁₅)alkylaryl represents an aryl having an (C₁-C₁₅)alkyl as a substituent. Note that the meanings of the (C₁-C₁₅)alkyl and aryl are the same as mentioned above. Preferable examples of the (C₁-C₁₅)alkylaryl include tolyl(2-methylphenyl, 3-methylphenyl, 4-methylphenyl), and 4-ethylphenyl, xylyl (for example, 2,4-dimethylphenyl, 2,5-dimethylphenyl, 3,5-dimethylphenyl, and 3,4-dimethylphenyl).

In the present invention, -(CₙH₂ₙ)R (where n is an integer of 1 to 15) represents a group (-CH₂CH₂...CH₂CH₂R) in which R is bonded to the carbon atom of the end of (CH₂)ₙ or a group (-CH₂CH₂...CHRCH₃) in which R is bonded to a carbon atom except the carbon atom at the end. R represents COOR' which substitutes for any one of n carbon atoms, and R' is H or (C₁-C₁₅) alkyl. The meaning of the (C₁-C₁₅) alkyl in R' is the same as mentioned above. Preferable examples of - (CₙH₂ₙ)R include -CH₂COOH, - (CH₂)₂COOH, -(CH₂)₃COOH, - (CH₂)₄COOH, and -CH₂COOCH₃.

In the present invention, the meaning of (C₁-C₁₅) alkyl in the (C₁-C₁₅) alkylcarbonyl is the same as mentioned above. Preferable examples of (C₁-C₁₅) alkylcarbonyl are -COCH₃ and -COCH₂CH₃.

In the present invention, the meanings of aryl in arylcarbonyl is the same as mentioned above. Preferable examples of arylcarbonyl are benzoyl and 1-naphtoyl.

In the present invention, the meanings of aryl and (C₁-C₁₅)alkyl used in the aryl(C₁-C₁₅)alkyl are the same as mentioned above. Preferable examples of aryl(C₁-C₁₅)alkyl are benzyl and phenethyl.

In the present invention, the meanings of aryl and (C₁-C₁₅) alkyl used in the aryl (C₁-C₁₅)alkylcarbonyl are the same as mentioned above. Preferable examples of aryl(C₁-C₁₅)alkylcarbonyl are benzylcarbonyl and phenethylcarbonyl.

In the present invention, the most preferable -C(=O)(CH₂)₁₋₅COOH is -C(=O)(CH₂)₁₋₆COOH. For example, -C(=O)CH₂COOH and -C(=O)(CH₂)₂COOH may be mentioned.

In the present invention, 2-pyridylcarbonyl, nicotinoyl(3-pyridyl carbonyl), isonicotinoyl(4-pyridylcarbonyl), toluene sulfonyl, carbamoyl, and amino are groups all preferably used.

In the present invention, the (C₁-C₆) alkyl in the carbamoyl having a substituent of one or two (C₁-C₆) alkyls, is preferably methyl, ethyl, and n-propyl. Preferable examples include methyl carbamoyl, dimethyl carbamoyl, ethyl carbamoyl, and diethyl carbamoyl.

In the present invention, the (C₁-C₆) alkyl in the amino substituted by one or two (C₁-C₆) alkyls, methyl, ethyl, and n-propyl are particularly preferable. Preferable examples include methyl amino, dimethyl amino, ethyl amino, and diethyl amino.

In the present invention, the meaning of aryl in the aryloxy optionally having a halogen on the ring is the same as mentioned above. The halogen refers to fluoro (F), chloro(Cl), bromo (Br), and iode (I). Of them, chloro is particularly preferable. Preferable examples of aryloxy optionally having a halogen on the ring include phenoxy methyl, phenoxy ethyl and 4-chlorophenoxy methyl.

In the present invention, preferable examples of the aryl having a (C₁-C₆) alkoxy or (C₁-C₆) alkoxycarbonyl are 4-methoxyphenyl and 4-acetylphenyl

As used herein, the term "ascofuranone homologue" means a compound having an analogous structure as that of ascofuranone, unless otherwise specified, and refers to a compound having at least an olcyl aldehyde moiety having an appropriate side chain, for example, terpenoid, at the 3-position of the olsyl aldehyde moiety and H or a halogen (eg., Cl) at the 5-position, and capable of binding to an intranuclear receptor PPAR. dehydroascofuranone is also included in the ascofuranone homologue.

In the present invention, the term "ascochlorin homologue" means a compound having an analogous structure as that of ascochlorin, unless otherwise specified, and refers to a compound having at least an olcyl aldehyde moiety, having an appropriate side chain, for example, terpenoid, at the 3-position of the olsyl aldehyde moiety and H or a halogen (eg., Cl) at the 5-position, and capable of binding to an intranuclear receptor PPAR.

Examples of the compound of the present invention include stereoisomers and pharmaceutically acceptable salts thereof. In the present invention, examples of the stereoisomers include geometrical isomers, tautomers, and optical isomers. Examples of the pharmaceutically acceptable salts include salts with an acid, such as chlorides, sulfates, nitrates, phosphates, hydrobromides, acetates, ascorbates, benzoates, salicylates, succinates, maleates, malates, and tartrates; and salts with a base, such as sodium salts, potassium salts, magnesium salts, and calcium salts.

Next, ascofuranone and homologues thereof as well as ascochlorin and homologues thereof will be described below.

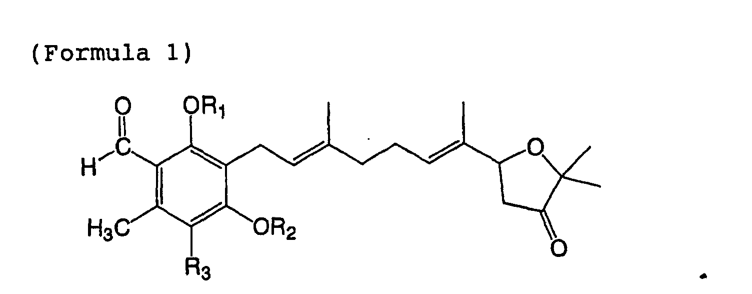

### (Compound represented by Formula 1)

| Compound No. | R₁ | R₂ | R₃ |
|---|---|---|---|
| 1 | H | H | Cl |
| 2 | H | -COCH₃ | Cl |
| 3 | H | -CH₃ | Cl |
| 4 | -CH₃ | -CH₃ | Cl |
| 5 | -COCH₃ | -CH₃ | Cl |
| 6 | -CH₃ | -COCH₃ | Cl |
| 7 | -CH₃ | H | Cl |
| 8 | H | - CH₂CH₃ | Cl |
| 9 | H | allyl | Cl |
| 10 | H | n-butyl | Cl |
| 11 | H | -CH₂COOH | Cl |
| 12 | H | -CH₂COOCH₃ | Cl |
| 13 | H | nicotinoyl | Cl |
| 14 | H | isonicotinoyl | Cl |
| 15 | H | -CO(CH₂)₃COOH | Cl |

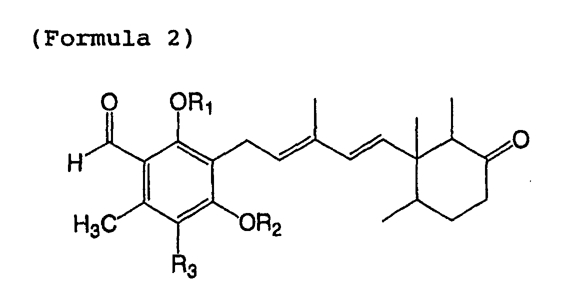

### (Compound represented by Formula 2)

| Compound No. | R₁ | R₂ | R₃ |
|---|---|---|---|
| 16 | H | H | Cl |
| 17 | H | -CH₃ | Cl |
| 18 | -CH₃ | -CH₃ | Cl |
| 19 | -COCH₃ | -CH₃ | Cl |
| 20 | -CH₃ | -COCH₃ | Cl |
| 21 | -CH₃ | H | Cl |
| 22 | H | -CH₂CH₃ | Cl |
| 23 | H | allyl | Cl |
| 24 | H | n-butyl | Cl |
| 25 | H | -CH₂COOH | Cl |
| 26 | H | nicotinoyl | Cl |
| 27 | -CH₃ | -CH₂COOH | Cl |
| 28 | -CH₃ | -CH₂COOCH₂CH₃ | Cl |
| 29 | H | -CO(CH₂)₂COOH | Cl |
| 30 | H | isonicotinoyl | Cl |
| 31 | -CH₃ | isonicotinoyl | Cl |
| 32 | H | H | Cl |
| 33 | H | -CH₃ | Cl |
| 34 | H | -COCH₃ | Cl |

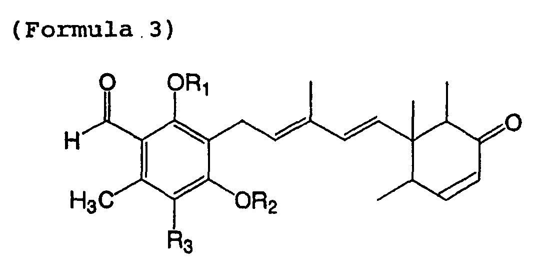

### (Compound represented by Formula 3)

| Compound No. | R₁ | R₂ | R₃ |
|---|---|---|---|
| 35 | H | H | H |
| 36 | H | -CH₃ | Cl |
| 37 | H | -COCH₃ | Cl |
| 38 | H | -CH₃ | H |
| 39 | H | -COCH₃ | H |

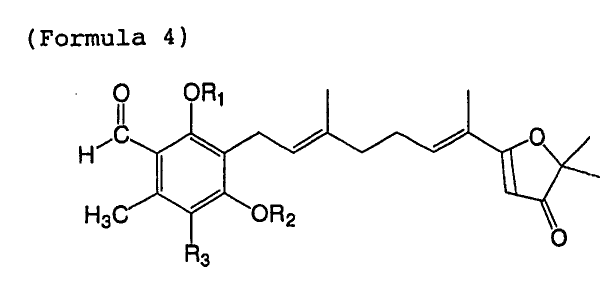

### (Compound represented by Formula 4)

| Compound No. | R₁ | R₂ | R₃ |
|---|---|---|---|
| 40 | H | H | Cl |
| 41 | H | -COCH₃ | Cl |
| 42 | H | -CH₃ | Cl |
| 43 | -CH₃ | -CH₃ | Cl |
| 44 | -COCH₃ | -CH₃ | Cl |
| 45 | -CH₃ | -COCH₃ | Cl |
| 46 | -CH₃ | H | Cl |
| 47 | H | -CH₂CH₃ | Cl |
| 48 | H | allyl | Cl |
| 49 | H | n-butyl | Cl |
| 50 | H | -CH₂COOH | Cl |
| 51 | H | -CH₂COOCH₃ | Cl |
| 52 | H | nicotinoyl | Cl |
| 53 | H | isonicotinoyl | Cl |
| 54 | H | -CO(CH₂)₃COOH | Cl |

### Manufacturing process

The compounds of the present invention are roughly classified to metabolic products, ascochlorin and ascofuranone produced by culturing a filamentous fungus, such as Ascochyta viciae, and their homologues, and their derivatives modified in a manner of organic-chemistry. Ascofuranone is an antibiotics (Tetrahydron Letters NO. 25, 2541-2544) produced by a filamentous fungus, Ascochyta viciae as reported by one of the present inventors in 1972. This filamentous fungus produces ascofuranone and ascochlorin at the same time. On the other hand, ascochlorin has been reported to be produced by filamentous fungi other than Ascochyta viciae, such as Nectria coccines, Fusarium so., Cylindocarpon lucidium, Cylindrocladium ilicicola, cylindrocladium sp., and Verticillium sp.; however, there is no reports that ascofuranone is isolated from these filamentous fungi. Ascofuranone is a metabolic product specific to Ascochyta viciae.

Dehydroascofuranone can be synthesized from ascofuranone as a starting material and specifically from a tetrahydrofuran ring by performing a dehydrogenation reaction in accordance with a conventional method. Furthermore, acylated or alkylated dehydroascochlorin can be synthesized by the following method. Note that this method can be also used in acylating or alkylating ascochlorin, ascofuranone and derivatives thereof.

Optical isomer of the compound of the present application can be manufactured by a known customary means (optical separation or chromatographic separation or asymmetric synthesis).

### Acylation

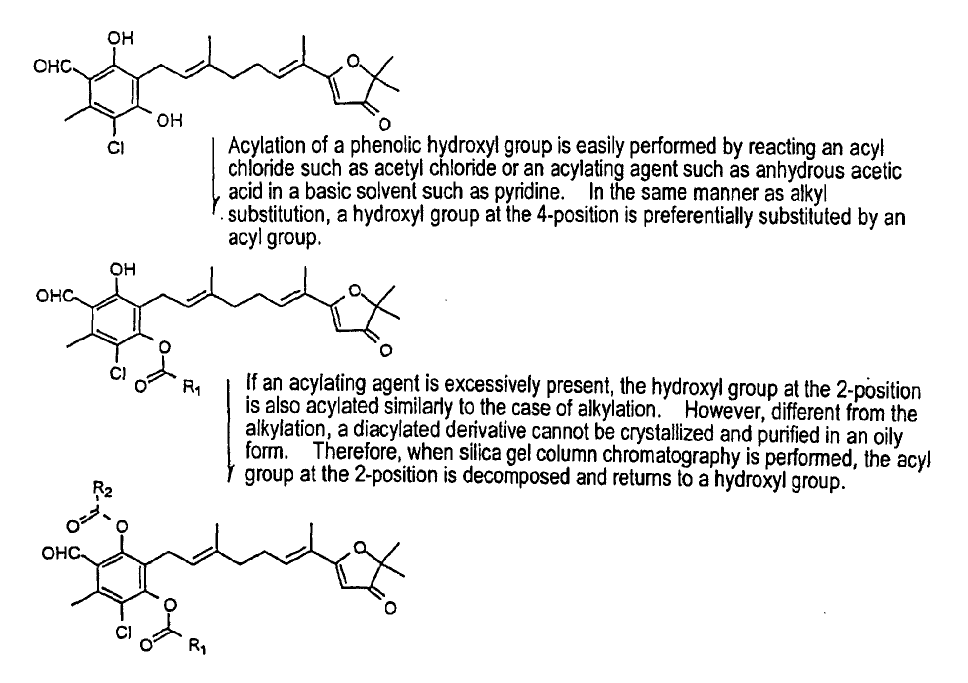

### Alkylation

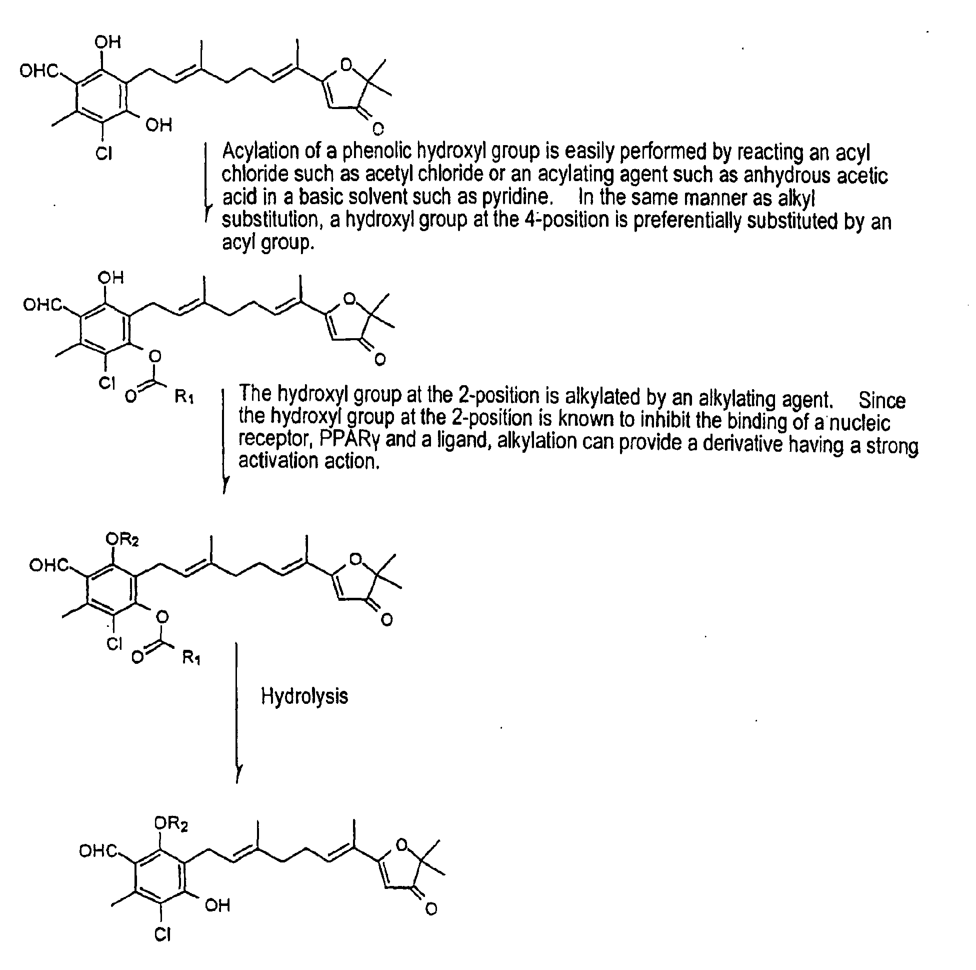

### 2-O-alkyldehydroascofuranone derivative

The hydroxyl group at the 2-position of olsyl aldehyde is substituted by an alkyl group as follows:

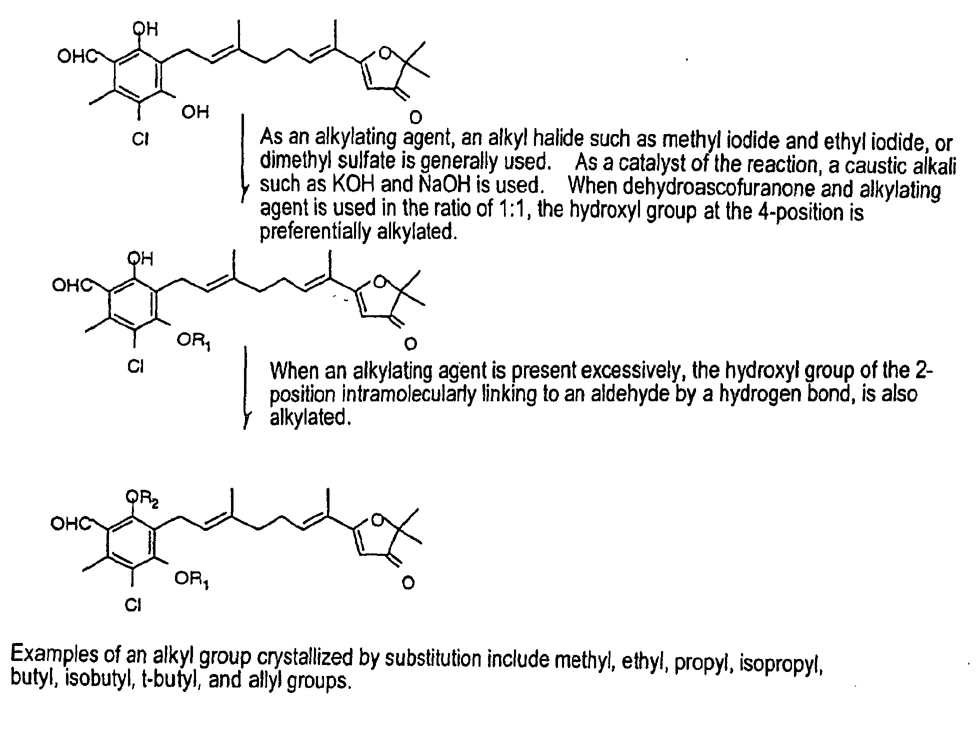

### Function

### [Results of animal test]

Test results of compounds of this series examined in various types of model animals such as mice, rats, dogs, quails, and rabbits are shown in Table 2.

Compounds of this series has an antihypertensive effect in various types of test animal models. The antihypertensive effect is confirmed not only in an acute test, in which arterial blood pressure of anesthetized rats and dogs is measured, but also in a chronic test using DOCA hypertensive rats, which are prepared by excising one of the kidneys and administering mineral corticoid and saline thereto, naturally-occurring hypertensive rats, which is an animal morbid model of essential hypertensive disease, and ob/ob mice, which are hereditary diabetic obese rats.

Compounds of this series is capable of lowering total serum cholesterol significantly when it is perorally administered to healthy animals such as mice, rats, and beagle dogs. It should be particularly notable that the compound significantly suppresses an increase of total serum cholesterol even in animal morbid models of hypercholesteremia, such as quails and rats. The quails are raised by giving a feed containing 2% cholesterol for 9 months to develop hypercholesteremia, and, in the case of the rats, mineral corticoid and saline are administered to allow them to develop heart and blood vessel disease and then hypercholesteremia.

Hereditary diabetic obese rats, such as db/db mice, ob/ob mice and Zucker rats, exhibit neutral-fat hyperlipemia, which is so severe that the serum become clouded. Compounds of this series lowers the level of serum neutral fat of healthy animals such as mice, rats, and beagle dogs, whereas it significantly lowers the level of serum neutral fat of hereditary diabetic obese animals. The effect of the compound in lowering the level of serum neutral fat is so strong that it makes clouded serum transparent. Furthermore, it is confirmed that the compound has an effect of lowering a high-level serum free-fatty acid in diabetic animals.

On the other hand, compounds of this series exhibits various effects for bringing back abnormal sugar metabolisms to be normal in a hereditary diabetic obese mouse, for example, lowering blood sugar (glucose), improving glucose tolerance, lowering serum insulin, improving insulin sensitivity, improving polydipsia and polyurea, and reducing the level of excreted urine sugar (glucose).

As to prevention/therapy of arteriosclerosis, a big difference is observed in effect between medicaments conceivably effective in improving arteriosclerosis, such as fibrate series medicament, thiazolidine series medicament, probucol, and statin series medicament and a compound of the present invention. The biggest difference between them resides in that the former medicaments do not exhibit anti-inflammatory effect on inflammatory lesion of the artery, whereas the latter medicament exhibits direct anti-inflammatory effect, as is suggested by accumulated test results. To explain more specifically, compounds of this series exhibits an effect of significantly inhibiting arteriosclerosis of rabbits and quails, which is induced by giving feed containing a large amount of lipid. Furthermore, the compound of this series exhibits an effect of preventing hypertension, hypertrophy of the heart, thickening of the artery, and edema of the kidney, which are developed in a rat having one of the kidneys excised out by administering mineral corticoid to the rat and raising it while feeding saline as drinking water.

Rats having a cardiovascular disease due to hypertension, often develop periarteritis nodosa, which is typical blood-vessel inflammation caused in thin and small artery such as mesenteric artery. Compounds of this series significantly inhibits onset of periarteritis nodosa. These are preventive effects. However, interestingly, compounds of this series exhibit therapeutic effects to periarteritis nodosa. To explain more specifically, mineral corticoid is administered to rats having one of the kidneys excised out and raised for a month by giving saline as drinking water. After confirming onset of periarteritis nodosa, administration of mineral corticoid and saline is stopped and the rats are brought back under normal feeding conditions. As a result, the blood pressure of the rats gradually decreases near a normal value and periarteritis nodosa heals and disappears. At that time, when compounds of this series is perorally administered to the rats, the healing and disappearance of periarteritis nodosa are significantly accelerated.

In the multiple risk factor syndrome, upper-body obesity, that is, a large amount of fat deposition onto the abdomen (visceral fat syndrome) is the most outstanding risk signal. According to recent studies, glucocorticoid produced by the adrenal cortex is suspected as a culprit causing visceral fat syndrome. This hypothesis basically come from the fact: first, lipid is significantly deposited onto the abdomen of a patient of cushing's syndrome, which is induced by excessive glucocorticoid produced because the adrenal cortex is excessively produced. Second, a patient administered with glucocorticoid for disease treatment has upper-body obesity. At present, it is confirmed that excessive production of an enzyme for producing glucocorticoid in the adrenal cortex is a cause of accelerating lipid deposition onto the abdomen, from analysis performed in a transgenic mouse. Therefore, control of in-vivo glucocorticoid production may become critically important to prevent and treat multiple risk factor syndrome.

Compounds of this series suppresses hepatic glucogenesis which is induced by administration of dexamethazon to a rat. For this reason, it antagonizes the action of glucocorticoid in vivo. The compound further has an effect of suppressing steroid diabetes characterized by polydipsia and polyurea and urine sugar excretion induced by administering cortisol to a rat. Furthermore, it is known that 4-O-methylascochlorin labeled with an isotope is widely and specifically distributed in the adipose tissue as well as in the adrenal gland and regulates synthesis of steroid hormones (glucocorticoid+mineral corticoid) of adrenal cortex. These facts strongly support that compounds of this series is useful in preventing and/or treating visceral fat syndrome.

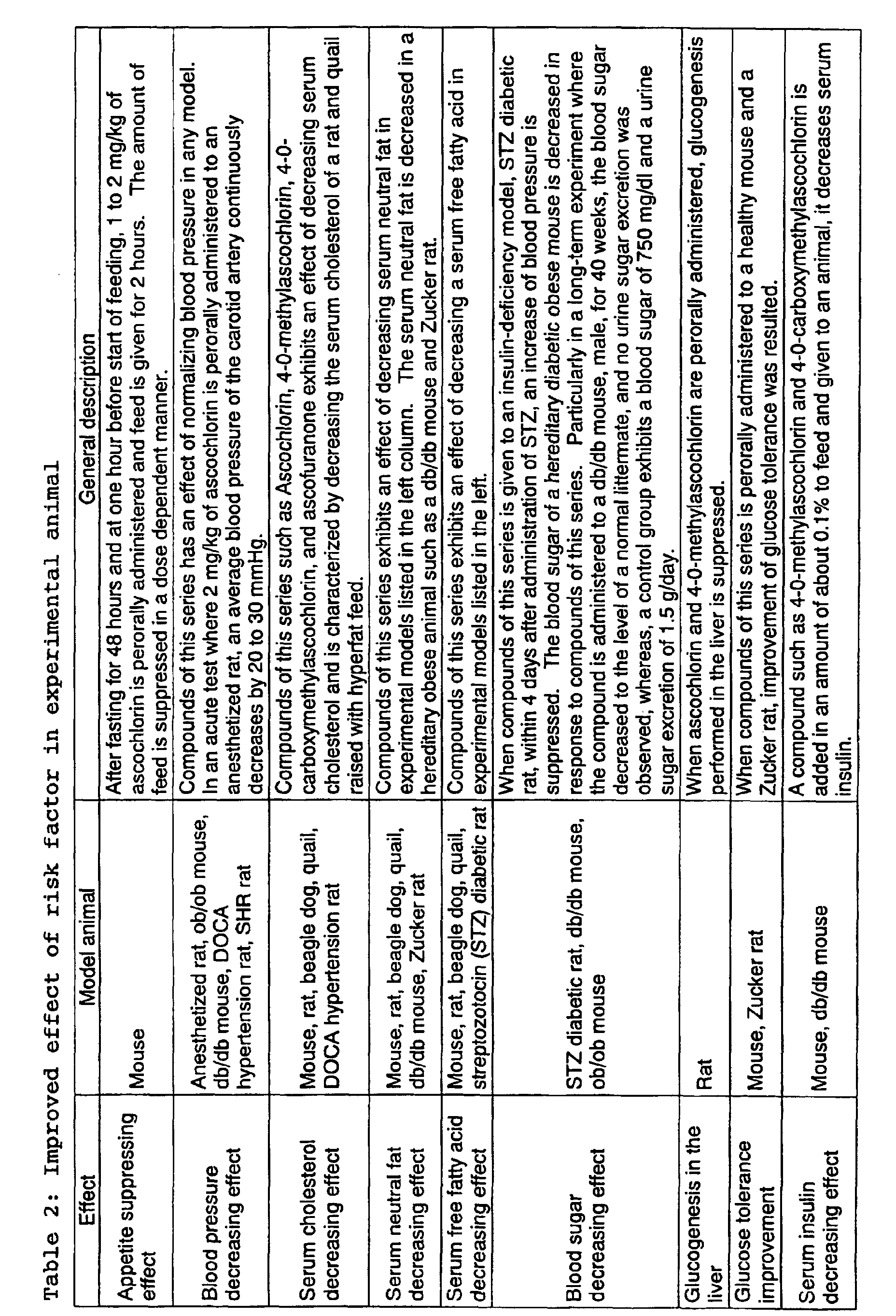

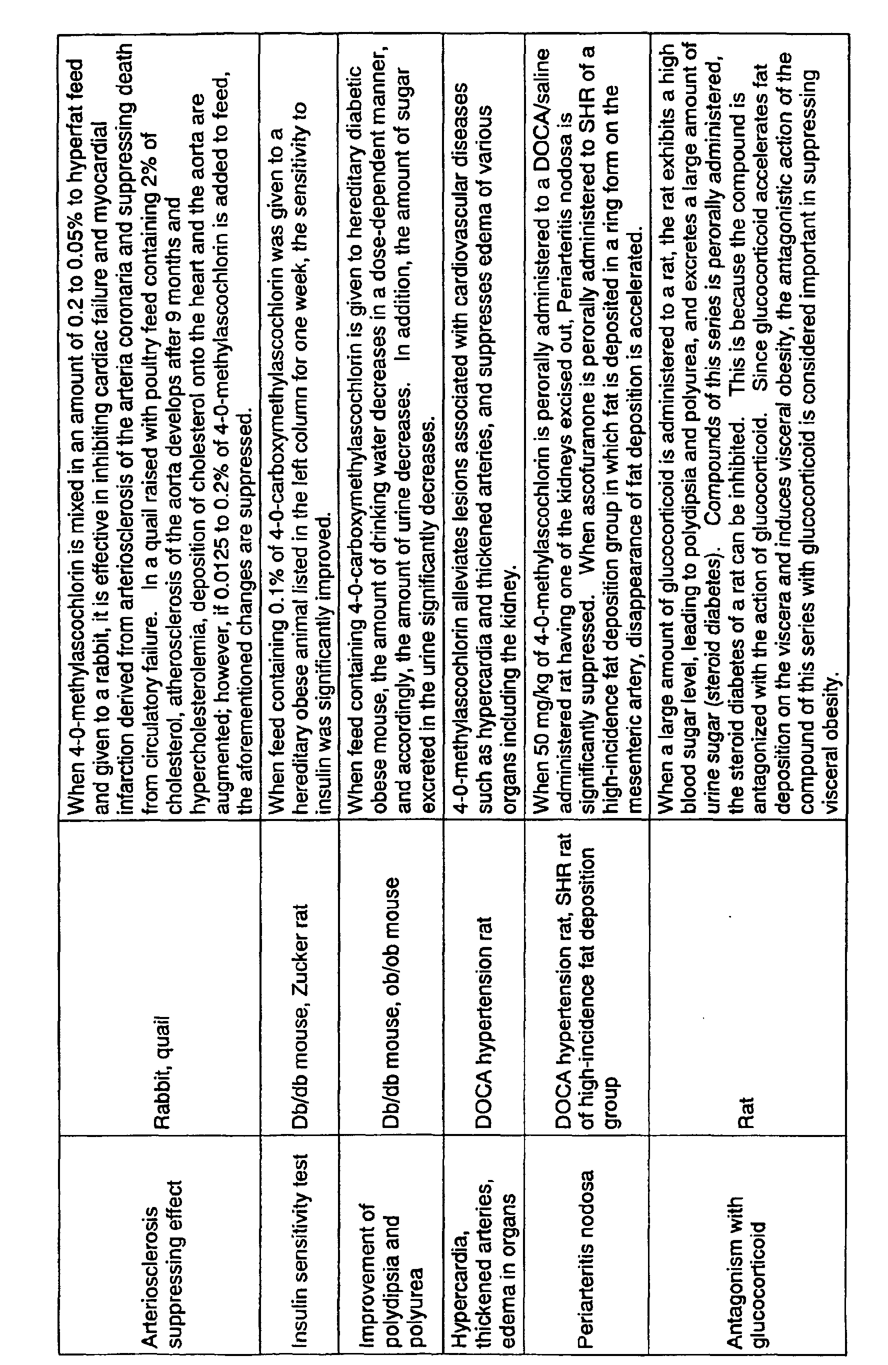

### [Results of clinical test]

On the other hand, the results of a clinical test using compounds of this series are summarized in Table 3 below. Clinical test I was performed in a group of 16 participants from hypercholesteremia patients who had a complication of hypertension and diabetes. On the other hand, Clinical test II was performed in a group of 79 participants from non-insulin dependent diabetic patients.

**Table 3:**

| Effect of improving risk factor in clinical test | | |
|---|---|---|
| Item | | Summary of results |
| Body weight | Clinical test I | Slight but significant decrease |
| | Clinical test II, first half period | Significant decrease in patients under dietetic therapy |
| Diastolic pressure | Clinical test I | Decrease to the lower limit value of normal range |
| | Clinical test II, first half period | Significant decrease in patients not responsive to administration of an antihypertensive agent |
| Systolic pressure | Clinical test I | Significant decrease |
| | Clinical test II, first half period | Decrease to the lower limit value of normal range |
| Serum cholesterol | Clinical test I | Significant decrease |
| | Clinical test II, first half period | Significant decrease, which is highly related to body weight decrease |
| Serum neutral fat | Clinical test I | Neutral fat of LDL decreases but VLDL is non-responsive |
| | Clinical test II, first half period | Significant decrease in neural-fat hyperlipemia |
| Blood sugar decreasing effect | Clinical test I | Significant decrease |
| | Clinical test II, first half period | Ditto |
| Serum free fatty acid | Clinical test I | Significant decrease |
| | Clinical test II, first half period | Not measured |
| Glucose tolerance improvement | Clinical test I | Not measured |
| | Clinical test II, first half period | Significant improvement |

As is described in detail in Examples, the effect of compounds of this series in improving risk factors of multiple risk factor syndrome patients can be demonstrated based on the results of Clinical test I performed in a group of 16 hypercholesteremia patients and Clinical test, second phase, a first half period, performed in a group of 79 type II diabetic patients.

### Preparation of pharmaceutical product using compounds of this series

A compound of the present invention is administered in the form of a pharmaceutical agent used in an analogous application, and more specifically, administered as a pure product or an appropriate pharmaceutical composition by way of an optional administration route. Therefore, administration can be performed in a dosage form of a solid matter, semisolid matter, lyophilized powder, or liquid such as a tablet, suppository, pill, capsule, powder, liquid, suspension, emulsion, cream, lotion, aerosol, ointment, and gel, and perorally, pernasally, parenterally, or locally, and preferably in a unit dosage form capable of administering an accurate dose per time. Such a composition contains a general pharmaceutical carrier, excipient, and a compound of the present invention, and optionally may contain another pharmaceutical product for medical use, a carrier, an absorption aid and the like. In general, a pharmaceutically acceptable composition contains about 1 to 99 % by weight of a compound of the present invention and an appropriate pharmaceutical additives about 99 to 1% by weight. These ratios vary depending upon an administration dosage form. This composition contains about 5 to 75% of a compound of the present invention as a pharmaceutical product for medical use and the reminder being an appropriate excipient for medical use.

The dosage per day effective for improving a pathological state is 0.1 to 20 mg/kg in terms of adult body weight and desirably 0.2 to 5 mg/kg. A pharmaceutical agent for treating the diseases mentioned above is preferably prepared in such a dosage form that a dose can be selected depending upon the severity of the disease to be treated. The most important point taken care in preparing a pharmaceutical agent is a limitation derived from lipid solubility of the chemical compounds of the present invention. Since the ligand of intranuclear receptor/superfamily is a lipid-soluble hormone or vitamin, a compound of the present invention must be lipid-soluble, as a natural consequence.

A pharmaceutically acceptable additive for oral administration is prepared by adding an optional excipient conventionally used, such as mannite, lactose, starch, magnesium stearate, sodium saccharin, talk, cellulose, glucose, gelatin, sucrose, or magnesium carbonate. Such a composition takes a form of liquid, tablet, pill, capsule, powder or sustained release preparation, and more preferably takes a tablet or pill. Such a composition contains a diluent such as lactose, sucrose, and dicalcium-phosphate; disintegrating agent such as starch and a derivative thereof; a lubricant such as magnesium stearate; a binder such as Arabia gum, polypinyl pyrrolidone, gelatin, cellulose and derivatives thereof; a surfactant which has a function of moistening the surface of particles of a compound of the present invention, which is highly lipid soluble and water-repellant, with water; a lipid soluble additive; bile acid; and phosphor lipid. It is particularly preferable to contain an aliphatic synthetic surfactant or a polymer assistant soluble in an organic solvent. Examples of these include Arabia gum, sodium alginate, methylcellulose, carboxymethyl cellulose, hydroxypropyl cellulose, polyvinyl pyrrolidone, bentonite, sodium lauryl sulfate, polysorbate 80, sorbitan monofatty acid ester, and polyoxyl stearate 40.

A particularly important point in preparing a pharmaceutical agent is a limitation due to physiological property being water repellant of a compound of the present invention. Generally, absorption of a lipid soluble medicament through the gastrointestinal tract is restricted by the rate of a single molecule of a medicament particle into water. This is because if a lipid-soluble medicament is dissolved in water, it can be rapidly absorbed virtually in a barrier free fashion when it reaches an absorption site of the mucosa of the gastrointestinal tract. Even if a medicament hardly soluble in water but it can be dissolved in water at a high rate, it can be smoothly absorbed perorally. In this case, its bioavailability is high. Unfortunately, it is known that the dissolution rate of a compound of the present invention in water is extremely low. Hence, to prepare a pharmaceutical product, it is necessary to provide an additive for increasing a dissolution rate of the compound and require a special device for preparing a pharmaceutical agent.

In the case where a compound such as a compound of the present invention is hard-to-soluble in water and dissolved at an extremely low rate, to facilitate the dissolution of a compound in water, it is considered to reduce the diameter of medicament particles as much as possible. This means: (1) the diameter of particles is reduced in order to increase the surface area of particles in contact with water. However, even through the diameter of particles is reduced, if the surface of particles is water repellant, the particles cannot come into contact with water. In this case, the dissolution rate will not increase unless fine particles are dispersed. Therefore, to bring the surface of particles in contact with water, (2) a surfactant or a polymer having not only a lipid-soluble group but also a water-soluble group must be present together with a compound of the present invention. In addition, (3) a device is required for easily separating single molecules from medicament particles. To explain more specifically, when medicament particles are crystalline, it is preferable to choose a crystal form such that single molecules can be separated from the lattice of the crystal with low free energy. Besides this, the manufacturing step must be devised such that a compound of the present invention is converted into amorphous form from which single molecules are easily separated. These essential conditions for preparing a pharmaceutical agent using a compound of the invention are as described in the claims of a compound invention of the present invention.

Furthermore, the kit of the present invention contains a pharmaceutically composition comprising one or more compounds of ascofuranone and derivatives thereof as well as ascochlorin and derivatives thereof according to the present invention and at least one of various carriers shown in the above and an instruction manual.

### Examples

The present invention will be described in more detail by way of Preparation Examples and Preparation Examples, which should not be construed as limiting the present invention.

### Example 1 (Preparation Example 1)

Raw materials were mixed such that one tablet contained 63 mg of 4-O-methylascochlorin, 10 mg of sesquioleate sorbitan, 10 mg of powder silica gel, 40 mg of corn starch, 50 mg of L-hydroxypropyl cellulose (PO-30), 12 mg of hydroxypropyl cellulose-SL, 46 mg of microcrystalline cellulose, 10 mg of sodium carboxymethyl cellulose, 3 mg of calcium stearate, and 6 mg of talk, granulated in accordance with a conventional method, and formed into tablets.

### Example 2 (Preparation Example 2)

100 g of 4-O-methylascochlorin, 70 g of microcrystalline cellulose, 35 g of lactose, 70 g of sodium carboxymethyl cellulose, 70 g of corn starch, and 5 g of magnesium stearate were mixed and formed into tablets in accordance with a conventional method, thereby preparing tables each having 0.35 g.

### Example 3 (Preparation Example 3)

C57BL/6j-ob/ob mice, which were model animals of multiple risk factor syndrome, were raised for a week with powered feed in which an ascochlorin derivative, 4-0-carboxymethylascochlorin (AS-6), was mixed in a content of 0.1%. Thereafter, the weight, blood sugar, blood insulin, and blood neutral fat were measured.

**Table 4:**

| Effect of 4-0-carboxymethylascochlorin administered to ob/ob mice on body weight, blood sugar, blood insulin, and blood lipid of the mice (8 mice/group) | | | | | |
|---|---|---|---|---|---|
| ob/ob mice | | | | | |
| | | Lean control group | ob/ob control group | AS-6 administered group | |
| Body weight | (g) | 24.5±0.5 | 48.5±0.6 | 47.8±0.6 | ns |
| Blood sugar | (mg/dL) | 220.9±8.2 | 360.4±44.9 | 243.8±15.6 | -32.4%* |
| Insulin | (µU/mL9 | 7.2±2.0 | 73.9±16.7 | 48.9±8.2 | -33.8% |
| Cholesterol | (mg/dL) | 112.8±1.7 | 268.5±24.2 | 249.7±12.1 | ns |
| Free fatty acid | (mEq/L) | 0.693±0.045 | 1.224±0.072 | 0.690±0.078 | -43.6%** |
| Neutral fat | (mg/dL) | 57.3±7.3 | 162.4±16.0 | 63.2±7.2 | -61.0%** |

| | | | | | |
|---|---|---|---|---|---|
| Average value ± standard deviation *p<0.05, **p<0.01 ob/ob control group vs AS-6 administered group | | | | | |

Compared to a normal Lean littermate control group, the ob/ob control group exhibited significantly larger values with respect to body weight, blood sugar, blood insulin, and blood lipid. Although a plurality of risk factors were integrated in each of the ob/ob mice, the risk factors except for body weight and blood cholesterol were significantly reduced by AS-6 administration.

### Example 4

C57BL/Ksj-db/db mice, which were model animals of multiple risk factor syndrome, were raised for a week with powered feed containing an ascochlorin derivative, 4-0-carboxymethylascochlorin (AS-6), in a content of 0.1%. Thereafter, the weight, blood sugar, blood insulin, and blood neutral fat were measured.

**Table 5:**

| Effect of 4-0-carboxymethylascochlorin (AS-6) administered to db/db mice on body weight, blood sugar, blood insulin, and blood lipid of the mice (8 mice/group) | | | | | |
|---|---|---|---|---|---|
| db/db mice | | | | | |
| | | Lean control group | db/db control group | AS-6 administered group | |
| Body weight | (g) | 30.4±0.8 | 52.9±0.8 | 56.3±1.2 | ns |
| Blood sugar | (mg/dL) | 174.6±7.8 | 637.3±28.9 | 445.8±32.5 | -30.0%** |
| Insulin | (µU/mL) | 7.3±0.4 | 31.0±3.6 | 34.6±3.5 | ns |
| Cholesterol | (mg/dL) | 52.4±1.4 | 109.9±3.3 | 114.4±4.3 | ns |
| Free fatty acid | (mEq/L) | 0.764±0.07 | 1.164±0.03 | 0.918±0.1 | -21.1%** |
| Neutral fat | (mg/dL) | 147.8±3.8 | 378.5±19.3 | 223.5±17.7 | -38.3%** |
| Average value ± standard deviation **p<0.01 db/db control group vs AS-6 administered | | | | | |

Compared to a normal Lean littermate control group, the db/db control group exhibited significantly larger values with respect to body weight, blood sugar, blood insulin, and blood lipid. Although a plurality of risk factors were integrated in each of the db/db mice, blood sugar, blood free fatty acid, and blood neutral fat, all were significantly reduced by AS-6 administration.

### Example 5

Zucker rats were divided into two groups. One group was raised with commercially available feed to which 4-0-carboxymethylascochlorin (AS-6) was mixed up to a content of 0.12%. The other group and normal Lean-rat littermate group were raised for 10 weeks with the same commercially available feed containing no AS-6. Thereafter, body weight, white adipose cell weight, blood insulin, blood sugar, and blood lipid were measured (7 mice/group).

**Table 6:**

| Effect of 4-0-carboxymethylascochlorin administered to Zucker rats on the weight of white adipose cells, blood sugar, blood insulin, and blood lipid of the rats | | | | |
|---|---|---|---|---|
| | Lean control group | Zucker control group | AS-6 administered group | |
| Body weight | 171±10 | 362±13 | 358±15 | ns |
| White adipose | 0.33±0.03 | 4.06±0.09 | 3.66±0.11 | ns |
| cells | | | | |
| Blood sugar | 152.4±12.9 | 73.0±14.5 | 174.3±19.0 | ns |
| (full feeding) | | | | |
| Insulin | 25.5±2.3 | 176.3±14.7 | 135.1±15.6 | -23.4% |
| Cholesterol | 74.1±2.3 | 117.4±4.8 | 123.6±7.8 | ns |
| Free fatty acid | 6.609±0.038 | 1.391±0.110 | 0.696±0.069 | -50.0% |
| Neutral fat | 124.3±7.6 | 928.6±47.7 | 466.3±60.2 | -49.8% |
| Phospholipid | 179.6±4.1 | 340.8±12.0 | 292.1±8.9 | -14.3% |
| Average vatue ± standard deviation *p<0.05, **p<0.01 Zucker control group vs AS-6 administered | | | | |

Compared to a normal Lean littermate control group, the Zucker control group exhibited significantly larger values with respect to body weight, white adipose cells, blood sugar (full feeding), insulin, cholesterol, free fatty acid, neutral fat and phospholipid. Although a plurality of risk factors were integrated, insulin, free fatty acid, and neutral fat, and phospholipid were significantly reduced by AS-6 administration.

### Example 6

The effect of 4-0-carboxymethylascochlorin (AS-6) in a glucose tolerance test were checked by using Zucker rats. Zucker control rats, Zucker AS-6 administered rats (20 mg/kg, perorally for a week), and Lean control rats were fasted overnight. Next morning, blood was taken from the caudal vein, and thereafter, 2 g/kg of glucose was perorally administered. Blood was taken from the caudal vein 30, 60 and 120 minutes after the administration and checked for blood sugar (7 mice/group).

**Table 7:**

| Effect of AS-6 in glucose tolerance test using Zucker rats | | | | |
|---|---|---|---|---|
| | 0 minute after glucose loading | 30 minutes after glucose loading | 60 minutes after glucose loading | 120 minutes after glucose loading |
| Lean control group | 62.5±5.2 | 127.5±3.3 | 130.0±3.9 | 89.8±3.8 |
| Zucker control | 107.5±5.0 | 235.1±9.6 | 275.8±13.2 | 290.3±4.6 |
| group | | | | |
| | +72.0% | +84.4%** | +112.2%** | +225.1% |
| Zucker AS-6 | 86.3±4.5 | 167.5±3.8 | 220.2±5.0 | 202.8±3.6 |
| administered group | | | | |
| | - 19.7%(*) | -28.8%(**) | -20.2%(*) | -30.1%(**) |

| | | | | |
|---|---|---|---|---|
| Average ± standard deviation **p<0.01 Lean control group vs Zucker control group | | | | |
| (*) p<0.05 (**) p<0.01 Lean control group vs Zucker AS-6 administered group | | | | |

There was no significant difference in blood sugar level at full feeding between the Zucker rats and the Lean control group; however, the fasting blood sugar level of the Zucker rats was significantly high. The fasting blood sugar level was decreased by 19.7% by administration of AS-6.

In the glucose tolerant test, although a decrease of glucose tolerance was observed in the Zucker control group, significant decreases were observed in the blood sugar level 30, 60, 120 minutes after glucose loading by administration of AS-6.

### Example 7

Powered feed to which ascofuranone (AF) and ascochlorin (AC) as well as its derivatives, 4-0-methylascochlorin (MAC), 4-0-carboxymethylascochlorin (AS-6), and 4-0-nicotinoyl ascochlorin (AS-103) were added to feed at the proportions shown in the table, and the feed were given to db/db mice for a week and then analyzed for blood sugar decreasing effect (8 mice/group).

**Table 8:**

| Blood sugar decreasing effect in db/db mice | | | | | |
|---|---|---|---|---|---|
| | | Lean | | db/db | |
| | | Body weight (g) | Blood sugar level (mg/dL) | Body weight (g) | Blood sugar level (mg/dL) |
| Control | group | 20.9±0.8 | 211.2±7.8 | 44.6±0.9 | 549.8±21.8 |
| MAC | 0.05% | 21.3±0.9 | 223.1±11.6 | 45.5±1.8 | 478.0±28.3 |
| | | ns | ns | ns | -13.3%* |
| AS-103 | 0.05% | 18.4±0.3 | 181.8±7.6 | 42.0±1.9 | 331.3±45.2 |
| | | - 12.0%** | -13.9%* | ns | -39.7%** |
| AR-610 | 0.1% | 20.2±0.4 | 194.1±9.5 | 45.3±1.7 | 335.8±8.5 |
| | | ns | ns | ns | -38.9%** |
| AF | 0.4% | 21.8±0.4 | 161.1±8.3 | 44.8±1.4 | 385.5±40.9 |
| | | ns | -23.7%** | ns | -29.9%** |
| AC | 0.05% | 16.1±0.3 | 184.8±10.9 | 38.9±1.8 | 262.1±11.8 |
| | | - 23.0%** | -12.5%** | -12.8%** | -52.3%** |

| | | | | | |
|---|---|---|---|---|---|
| Average ± standard deviation *p<0.05, **p<0.01 | | | | | |

The blood sugar level of the db/db mouse group were 2.6 folds as high as that of the Lean control group. Blood sugar level was significantly reduced by administration of compounds of this series. Therefore, the effect of the compound in decreasing blood sugar was confirmed.

### Example 8

Zucker rats were raised for 4 weeks with feed in which 4-0-carboxylmethyl ascochlorin (AS-6) was mixed in a content of 0.1%. At the 0th, 2nd and 4th week of initiation of feeding, blood pressure was measured (6 rats/group).

**Table 9:**

| Effect of AS-6 on blood pressure of Zucker rat | | | |
|---|---|---|---|
| | 0th week | 2nd week | 4th week |
| Lean control group | 123±2 | 118±4 | 124±6 |
| Zucker control group | 145±5 | 153±6 | 156±7 |
| | +17.9%** | +29.7%** | +25.8%** |
| AS-6 administered group | 144±6 | 132±5 | 125±6 |
| | ns | -13.7%(**) | -19.9%(**) |

| | | | |
|---|---|---|---|
| Average ± standard deviation ** p<0.01 Lean control group vs Zucker control group | | | |
| (**) p<0.01 Zucker control group vs Zucker AR-610 administered group | | | |

In Zucker rats (10 week old), a significant increase in blood pressure was observed compared to its normal littermates (+ 17.9%). A decrease of 13.7% at the 2nd week and a decrease of 19.9% in blood pressure at the 4th week after the initiation of feeding were observed by administering AS-610 in comparison with the Zucker control group.

### Example 9

### Protocol of Clinical test I and the background of participants

The protocol of Clinical test I performed with 16 participants of hypercholesterolemia patients is as shown in the table. The participants consisted of 9 Caucasian males and 7 Caucasian females. They participated in Clinical test I on a voluntary basis after they signed informed consent for clinical test participation, which was proposed by a medical institution based on the Helsinki declaration.

Their ages were 55 years old in average and ranging from 40 to 82 years old. Their average body weigh was 70.2 ± 13.57 kg (standard deviation).

**Table 10:**

| Protocol of Clinical test 1 | | |
|---|---|---|
| Item | | |
| Test medicament | 4-O-methylscochlorin | |
| Test period | 12 weeks, a medicament is given for first 8 weeks and the following 4 weeks are a placebo period | |
| Participant Qualification | Male and female adults excluding women capable of pregnancy, out-patients with hypercholesterolemia. All are cases with out-patients | |
| Dose | Three times per day, intake after meals | |
| Dosage form | Hard capsule | |
| Medication schedule | 0-1st week | 0.5mg/kg (12 mg x 3) |
| | 1-2nd week | 1.0mg/kg (25 mg x 3) |
| | 2-3rd week | 2.0mg/kg (50 mg x 3) |
| | 3-4th week | 4.0mg/kg (100 mg x 3) |
| | 4-6th week | 8.0mg/kg (200 mg x 3) |
| | 6-8th week | 16.0 mg/kg (400 mg x 3) |
| | 8-10th week | 0.0 mg/kg (Placebo) |
| | 10-12th week | 0.0 mg/kg (Placebo) |
| Doctor's question | Check-up time, immediately before medication, and 1, 2, 3, 4, 6, 8, 10 and 12th week | |
| Blood pressure measurement | Immediately before medication, 1, 2, 3, 4, 6, 8, 10, and 12th week | |
| Electrocardiogram measurement | Immediately before medication, 8 and 12th week | |
| Serum biological test | Immediately before medication, 1, 2, 3, 4, 6, 8, 10, 12th week. Items: total cholesterol, neutral fat, free fatty acid, blood sugar, GOT, GPT, ALP, BUN, bilirubin, total protein, hemoglobin, etc. | |
| Hematocyte test | Immediately before medication. 1, 2, 3, 4, 6, 8, 10 and 12th week. Items: erythrocytes, leukocytes, platelets, and leukocyte percentage | |
| Urine test | Immediately before medication, 1, 2, 3, 4, 6, 8, 10, and 12th week | |
| Platelet aggregation test | Immediately before medication, 4, 8, and 12th week, ADP aggregation of platelet-rich plasma measured by an aggregometer | |
| Body weight | Immediately before medication. 1, 2, 3, 4, 6, 8, 10 and 12th week | |

All participants took dietary therapy for treating hypercholesterolemia. Since various medicaments were administered, the period of 8 weeks, prior to Clinical test I, was provided to stop taking all pills, thereby washing out effects of medicaments. During the period of the Clinical test I, it was not allowed to take any other medicament together with a test medicament. Patients with type II diabetes were excluded from the participants; however, 5 patients with a high blood sugar level were included in the participants. Since they were not diagnosed as diabetes, they were allowed to participate in the test. Of 16 participants, 10 patients had hypertension and 4 patients had borderline hypertension defined by WHO. Only 2 patients had normal blood pressure.

### Summary of results of Clinical test I

Clinical test I was completed for 12 weeks without drop-out patients. As a side effect, one case with light eruption and one case with dizziness were reported; however, they were not so severe that medication continued. The former one was a transient symptom and disappeared soon. Therefore, the causal relation with medication was unclear. The latter one is a hypertensive patient and the patient's side effect appeared due to a decrease in blood pressure. No abnormality ascribed to medication was observed in the serum biological test, hematocyte test, and urine test. The serum total cholesterol was lowered by 30% or more by medication. Furthermore, blood pressure of the hypertension group and borderline hypertension group dropped to the upper limit of a normal range and tended to gradually increase during the placebo administration period after 8 weeks. In 5 cases of exhibiting high blood sugar, the blood sugar levels were brought back to normal levels by medication. The difference obtained in a paired t-test was statistically significant.

### Fluctuation of total serum cholesterol in Clinical test I

As shown in Table 11, the effects of the compound of the present invention in reducing serum total cholesterol is not remarkable; however, have a statistically high significant difference. One week after 0.5 mg/kg of a compound of the present invention was perorally administered, the total serum cholesterol dropped by 70 mg/dl in average. At the 6th week when 16 folds as large as the amount was administered, the total serum cholesterol decreased to the upper limit of the normal range. Furthermore, the total serum cholesterol increases little by little from 8 to 12 weeks in which a placebo was started to administer in place of a test medicament; however maintains at a significantly lower level than that before medication. The efficacy of the medicament lasts long, suggesting that this medicament is not a simple enzyme-inhibitor such as hydroxyl methyl glytaryl Co-A inhibitor.

**Table 11:**

| Effect of 4-O-methylascochlorin in decreasing total serum cholesterol in Clinical test I | | | |
|---|---|---|---|
| Time | Dose (mg/kg) | Total serum cholesterol (mg/dl) | Statistical significant difference(t)* |
| Before medication | - | 342.0±61.0 | - |
| 1st week | 0.5 | 272.6±62.7 | P<0.005 |
| 2nd week | 1.0 | 266.5±66.7 | P<0.002 |
| 3rd week | 2.0 | 268.7±66.0 | P<0.005 |
| 4th week | 4.0 | 254.2±65.7 | P<0.0005 |
| 6th week | 8.0 | 217.4±58.8 | P<0.0005 |
| 8th week | 16.0 | 229.5±61.9 | P<0.0005 |
| 10th week | Placebo | 255.9±57.6 | P<0.0005 |
| 12th week | Placebo | 264.0±57.5 | P<0.002 |
| 16 patients with hypercholesterolemia participated. Significant difference value in Paired t-test is (average ± standard deviation) | | | |

### Antihypertensive effect in Clinical test I

The antihypertensive effect of 4-O-methylascochlorin on 10 hypertensive patients is shown in Table 12. From this, it may not be said that this agent quickly reduces blood pressure of the hypertensive patients; however, the agent lowers the blood pressure of the systolic period and the diastolic period gradually with the passage of time. At the 8th week, the agent decreased both pressure values to normal levels. In the period of the 10 to 12th week during which a placebo is given in place of this agent, blood pressure tends to gradually increase; however, compared to the value before medication, the blood pressure is significant low. This suggests that the effect of this agent on blood pressure also lasts long.

Blood pressure values in the systolic period and diastolic period of 4 borderline hypertensive patients were totalized and statistically analyzed. The results are shown in Table 13. The blood pressure of the systolic period returned normal a week after initiation of medication, and the blood pressure of the systolic period returned normal 6 weeks after the initiation of medication. Besides this, in the period of 10 to 12 weeks during which a placebo is given in place of the medicament, the pressure retains at a normal level. It suggests that the antihypertensive effect lasts long. Note that, during the period of Clinical test I, 2 patients of normal blood pressure maintain their blood pressure at a normal level. It clearly demonstrates that this agent does not affect normal blood pressure.

**Table 12:**

| Antihypertensive effect of 4-O-methylascochlorin in Clinical test I (Hypertension group) | | | |
|---|---|---|---|
| Time | Dose (mg/kg) | Systolic pressure (mmHg) | Diastolic pressure (mmHg) |
| Before medication | - | 161.5±21.1 | 91.0±7.7 |
| 1st week | 0.5 | 143.5±28.5* | 83.5±4.7* |
| 2nd week | 1.0 | 147.5±28.5* | 83.0±5.4* |
| 3rd week | 2.0 | 147.5±26.1* | 84.5±6.0* |
| 4th week | 4.0 | 148.5±26.8** | 85.5±9.44** |
| 6th week | 8.0 | 144.5±29.8** | 83.5±9.44* |
| 8th week | 16.0 | 130.0±7.6** | 78.1±3.7** |
| 10th week | Placebo | 145.5±26.3** | 83.5±8.8** |
| 12th week | Placebo | 151.5±32.4 | 84.0±9.7** |
| 10 patients having a systolic pressure of > 140 mmHg and a diastolic pressure of > 80 mmHg before medication are totalized. Numerical value is (average ± standard deviation) *P < 0.05, **P < 0.01 Significant difference in Paired t test. | | | |

**Table 13:**

| Antihypertensive effect of 4-O-methylascochlorin in Clinical test I (Borderline hypertensive group) | | | |
|---|---|---|---|
| Time | Dose (mg/kg) | Systolic pressure (mmHg) | Diastolic pressure (mmHg) |
| Before medication | - | 149.3±7.9 | 87.1±2.7 |
| 1st week | 0.5 | 133.6±9.0** | 82.1±3.9* |
| 2nd week | 1.0 | 134.3±6.7** | 81.4±4.8* |
| 3rd week | 2.0 | 134.3±9.3** | 81.4±2.4** |
| 4th week | 4.0 | 130.7±8.9** | 80.7±3.5** |
| 6th week | 8.0 | 130.7±8.9** | 78.6±3.8** |
| 8th week | 16.0 | 130.0±8.2** | 77.9±3.9** |
| 10th week | Placebo | 130.7±9.3** | 78.6±4.8** |
| 12th week | Placebo | 132.1±10.4** | 78.6±4.8** |
| 4 borderline hypertensive patients having a systolic pressure of > 140 mmHg and a diastolic pressure of > 80 mmHg before administrate are totalized. Numerical value is (average ± standard deviation). *P<0.05, **P<0.01 Significant difference in Paired t test. | | | |

### Fluctuation of blood sugar level in Clinical test I

Fluctuation of blood sugar level in Clinical test I is shown in Table 14. Of the patients participated in this test, 5 patients exhibiting a fasting blood sugar (FBS) level over 100 mg/dl were chosen and observed with respect to change in blood sugar level with time. The blood sugar level decreases to 32.4 mg/dl actually at the first week. At 6th week when the dose is increased to 8 mg/kg, the sugar level completely returns to a normal level. Also after 10th week when a placebo is given in place of a test agent, the sugar level is significantly low compared to that before initiation of medication, suggesting that the effect of this agent on blood sugar level lasts long, similarly to the cases of hypercholesterolemia and hypertension.

**Table 14:**

| The effect of 4-0-methylascochlorin on reducing blood sugar level in Clinical test I | | | |
|---|---|---|---|
| Time | Dose (mg/kg) | Fasting blood sugar (FBS) level (mg/dl) | Statistical significant difference (t)* |
| Before medication | - | 132.4±16.0 | - |
| 1st week | 0.5 | 100.0±11.0 | P<0.005 |
| 2nd week | 1.0 | 102.0±4.0 | P<0.025 |
| 3rd week | 2.0 | 103.6±12.1 | P<0.025 |
| 4th week | 4.0 | 103.2±11.7 | P<0.05 |
| 6th week | 8.0 | 98.0±4.6 | P<0.025 |
| 8th week | 16.0 | 93.3±6.1 | P<0.025 |
| 10th week | Placebo | 93.5±6.4 | P<0.01 |
| 12th week | Placebo | 100.5±11.6 | P<0.005 |
| 5 patients having a blood sugar level of > 120 mg/dl before medication are totalized. Numerical value is (average ± standard deviation) Significant difference in Paired t test. | | | |

### Fluctuation of body weight in Clinical test I

The patients participated in Clinical test I previously had dietary advices on hypercholesterolemia by a nutritionist. During the test period, the quality and amount of meals were chosen freely by individual patients within the allowable range determined by dietary therapy. Dietary regulation was not particularly performed. However, compared to before medication, the body weight of 8 weeks after the initiation of medication slightly decreased by 0.9 kg, which had a statistical significance (P < 0.001). There were no patients who increased in body weight. Since no patients who reported anorexia or gastrointestinal symptoms during the medication, the decrease in body weight was not caused by a side effect. The compound of this series differs from thiazolidine series compound in that medication of this compound does not increase body weight at all but gradually and definitely decreased.

### Fluctuation of serum neutral fat and free fatty acid in Clinical test I

It was found that hypercholesterolemia patients can be divided into two groups depending upon the serum neutral fat of the patients satisfactorily responds to 4-O-methylascochlorin or not. More specifically, in the case where a ratio of serum neutral fat/serum total cholesterol is 1.2 or less (corresponding to hyperlipemia type IIa based on Frederickson's classification), the serum neutral fat significantly decreased in response to medication. In contrast, in the case where the ratio is 1.2 or more (corresponding to hyperlipemia type IIb and type IV based on Frederickson's classification), a significant decrease of serum neutral fat was not observed. As lipoprotein particles having a ratio of neutral fat/total cholesterol is 1.2 or less, low-density lipoprotein (LDL) is mentioned. Conversely, in the case where the ratio is greater than 1.2, very low density lipoprotein (VLDL) occupies a large proportion of the lipoprotein contained in the serum. Therefore, it is suggested that 4-O-methylascochlorin selectively decreases LDL, which is said to be the most significant risk factor to ischemia heart disease; however, the effect of 4-O-methylascochlorin in reducing serum lipoprotein (VLDL) rich in neutral fat, is weak.

### Example 10

### Protocol of Clinical test II and background of participants

79 non-insulin dependent type II diabetes patients whose blood sugar is well-controlled under dietary therapy (all were mongoloid Japanese) participated in Clinical test II. They were forbidden to use sulfonyl urea and biguanide agent which is a therapeutic agent for type II diabetes, and a fibrate series medicament, nicotinic acid, and probucol which is a serum fat decreasing agent; however use of an antihypertensive agent is not particularly prohibited. The protocol of the second-phase test is as shown in the table. They participated on a voluntary basis after they signed informed consent for clinical test participation, which was proposed by a medical institution based on the Helsinki declaration. The participants consisted of 26 males and 53 females. An average age at the sign-up time was 58 ± 9.9 years old (average value ± standard deviation), ranging from 37 to 81 years old. The body weight of patients was divided by standard body weight obtained from the standard body-weight table issued by Meiji life Insurance Company. This index was 1.24 ± 0.154 (average value ± standard deviation) at the sign-up time, meaning that there are many obese persons. After 12 weeks, the body weight index decreased to 1.18 ± 0.137(average value ± standard deviation).

**Table 15:**

| Protocol of Clinical Test II | |
|---|---|
| Item | Content |
| implementation period | Medication period is 12 weeks |
| Participation qualification | Male and female adult, women capable of pregnancy are excluded, 79 Patients with non-insulin dependent type II diabetes within 10 year history under dietary therapy. |
| Medicament and medication | Medicament is 4-O-methylascochlorin, 3 times per day, intake after a meal. |
| Dosage form | Plain tablet |
| Dose | 0-12th week 6 mg/kg (360 mg ×3) |
| Doctor's question | Checkup time, immediately before medication, 4, 8, 12th week |
| Blood-pressure measurement | Immediately before medication, 4, 8, 12th week |
| Electrocardiogram measurement | Immediately before medication, 12th week |
| Serum biological test | Immediately before medication, 4, 8, 12th week Items: total cholesterol, neutral fat, free fatty acid, blood sugar, GOT, GPT, ALP, BUN, bilirubin, total protein, hemoglobin, etc. |
| Hematocyte test | Immediately before medication, 4, 8, and 12th week, Items: erythrocytes, leukocytes, platelets, and leukocyte percentage |
| Urine test | Immediately before medication, 4, 8, and 12th week |
| Glucose tolerance | Immediately before medication, 12th week |
| Body weight | Immediately before medication, 1, 2, 3, 4, 6, 8, 10 and 12th week |

Of 79 patients, 7 patients had a complication with hypertension (all were taking an antihypertensive agent) and 14 patients with borderline hypertension defined by WHO (all were taking an antihypertensive agent). The number of patients having normal blood pressure was 58 (of them, 31 patients were taking an antihypertensive agent).

### Summary of the results of Clinical test II

All patients completed a 12-week medication course of Clinical test II without drop-out patients due to side effect. Two cases of light eruption were observed as side effect; however the eruption was too light to stop medication. The eruption was a transient symptom and disappeared within a week. Therefore, causal association with medicament was unknown. According to the serum biological test, hematocyte test and urine test, medication did not bring any abnormality. In a group exhibiting a total serum cholesterol value of greater than 220 mg/dl before medication, the total serum cholesterol value was significantly decreased by medication. Different from Clinical test I, also serum neutral fat was significantly decreased by medication. Although a reduction rate of blood sugar by medication was low; the reduction rate had a statistically high significant difference. On the other hand, the blood pressure values of a hypertensive group and a borderline hypertensive group were significantly decreased by medication. In the glucose tolerance tests performed before and after medication, glucose tolerance was improved by medication. As urine was taken at the same time as above and checked, the amount of sugar excreted in the urine was significantly improved. The body weight significantly decreased by 0.9 kg, compared to that before medication.

### Fluctuation of body weight, fasting blood sugar, total serum cholesterol, and serum neutral fat in Clinical test II

Fluctuation of body weight, fasting blood sugar, total serum cholesterol, and serum neutral fat was shown in the table. The participants of the second-phase test were patients diagnosed as non-insulin dependent type II diabetes but not selected because they had abnormal values in the indexes (except fasting blood sugar) shown in the table. Therefore, patients having normal body weight, total serum cholesterol, and serum neutral fat were also included in statistics.

Although statistic included data of patients having normal-range indexes, significant decreases of the body weight, fasting blood sugar, total serum cholesterol, and serum neutral fat were found. It was confirmed that they are significantly decreased by therapy. Since all risk factors including body weight, fasting blood sugar, total serum cholesterol, and serum neutral fat are improved significantly, it is clear that compounds of this series improves multiple risk factor syndrome.

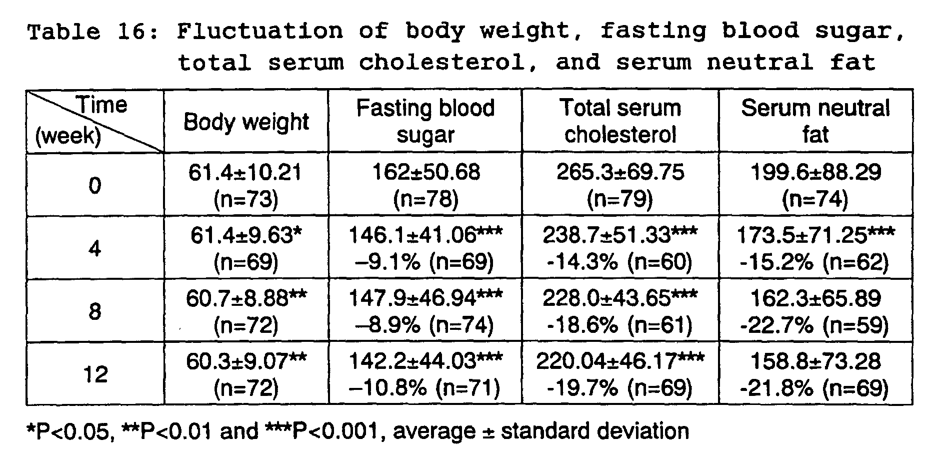

### Fluctuation of fasting blood sugar in Clinical test II

As is apparent from table 12 showing fluctuation of fasting blood sugar, 4-O-methylascochlorin does not have a blood sugar decreasing effect on a normal blood sugar group having a fasting blood sugar level of 130 mg/dl or less; however it has a significant blood sugar decreasing effect on a group having a fasting blood sugar level of greater than 130 mg/dl.

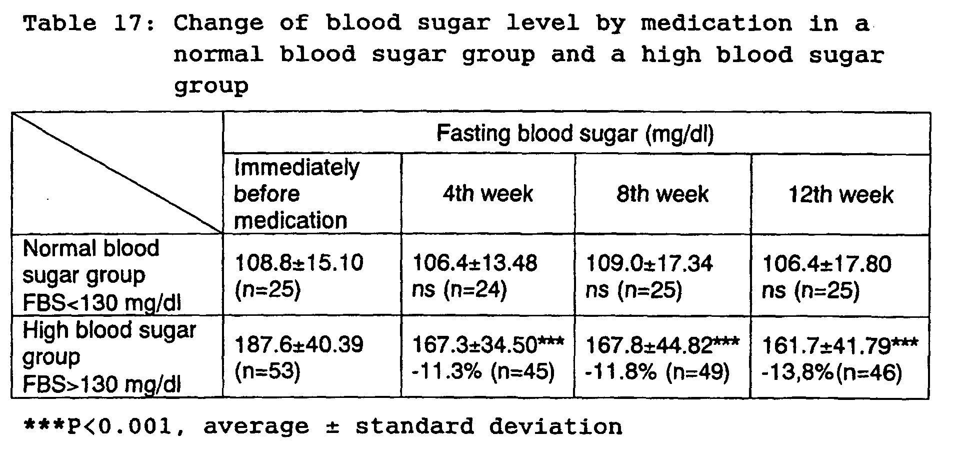

### Fluctuation of total serum cholesterol in Clinical test II

Type II diabetes patients participated in Clinical test II were classified into three groups: super high value group, high value group and normal group, in terms of concentration of serum total cholesterol.

Similarly to the case of fasting blood sugar, 4-O-methylaschochlorin did not change the concentration of serum cholesterol significantly in the normal group whose serum total cholesterol value was 220 mg/dl or less. However, in the super high value group and high value group, it was confirmed that the total cholesterol values were further significantly decreased by medication.

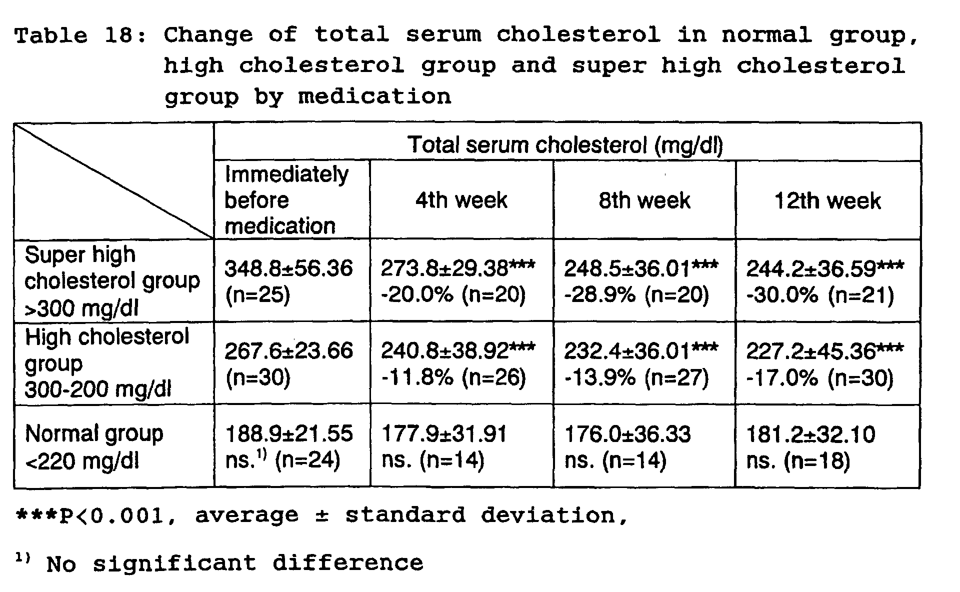

### Fluctuation of serum neutral fat in Clinical test II

Serum neutral fat concentration is an important index which is a risk factor defined in Syndrome X and the Deadly Quartet theory. Statistical operation was performed by dividing patients into a normal value group whose serum neutral fat fell within a normal range of 120 mg/dl or less, and an abnormally high value group whose serum neutral fat was greater than 120 mg/dl.

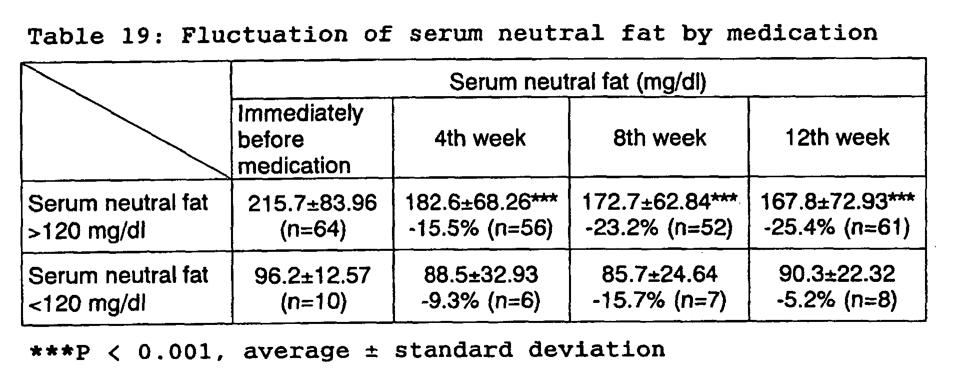

Similarly to the total serum cholesterol, the neutral fat of the abnormal high value group was significantly decreased by medication; however, that of the normal value group showed only a tendency to decrease.

### Fluctuation of blood pressure in Clinical test II

An antihypertensive agent was administered to about 66% of patients participated in Clinical test II. Despite of administration of the antihypertensive agent, 7 hypertension patients and 14 borderline hypertension patients were defined based on the WHO definition. Their data were subjected to statistical operation. The systolic pressure of the hypertension group decreased by 10 mmHg 4 weeks after medication. However, the decrease showed no significant difference. This was only one exception. The systolic pressure and diastolic pressure gradually and significantly decreased by therapy. To lower the blood pressure of the type II diabetes patients complicated by hypertension, an angiotensin-converting enzyme inhibitor, angiotensine II-antagonist, calcium antagonist and the like are used. Therefore, the results suggest that even if these antihypertensive agents often used are ineffective, compounds of this series exhibits an antihypertensive effect. The possibility that compounds of this series can greatly advance hypertension treatment was confirmed.

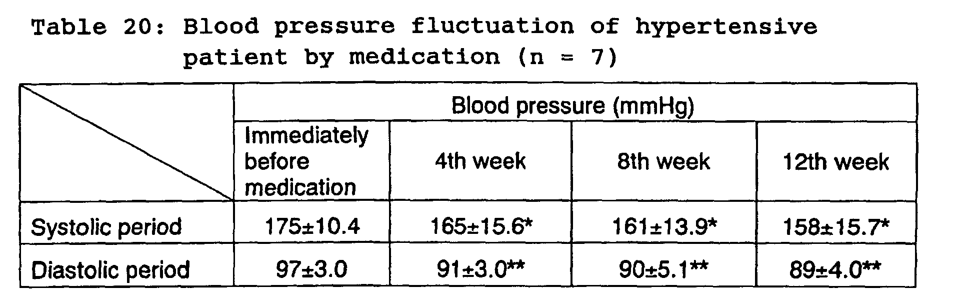

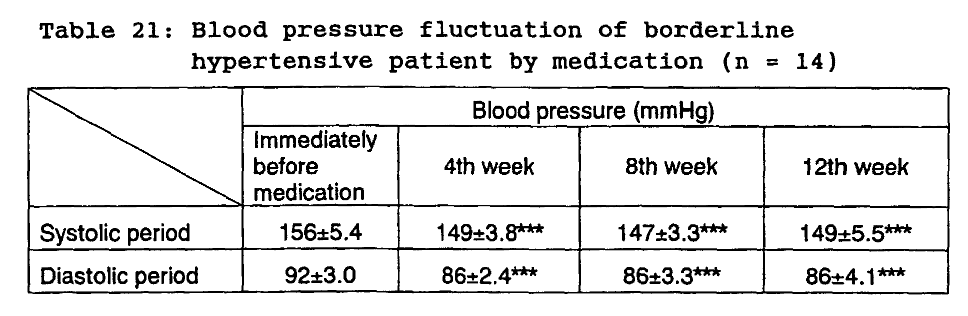

In the case of patients of type II diabetes complicated with borderline hypertensive, 4-0-methylascochlorin exhibited highly significant antihypertensive effect. These results means: compounds of this series do not prevent the effects of convenient antihypertensive agents; and if the conventional antihypertensive agents are ineffective, compounds of this series show antihypertensive effects. The results therefore suggest that compounds of this series can also be used alone as antihypertensive agents.

### The relationship between body-weight fluctuation and decreases of fasting blood sugar and total serum cholesterol in Clinical test II

As previously mentioned, when the sensitivity to insulin is low as the case of type II diabetes, obesity becomes a high risk factor. Then, the relationship between decrease of body weight, fasting blood sugar and total serum cholesterol was investigated. 79 patients were classified into three groups: a group of patients whose body weight "decreased by 3 kg or more", a group of patients whose body weight "decreased by 0.1 to 2.9 kg, and a group of patients whose body weight "did not change or increased" during phase II, first half test period of 12 weeks. Then, the relationship between fluctuation of body weight, fasting blood sugar and total serum cholesterol was computationally determined by Kruskal-Wallis Hc test. As a result, it has become clear that the decrease in body weight is significantly correlated to a decrease rate in fasting blood pressure with a significance level of 5% or less, as is shown in the table. This means that if the body weight decreases by 3 kg or more during a treatment period, the effect of lowering fasting blood pressure is definitely obtained. The results coincide with a conventional view that improvement of type II diabetes resides in treating obesity.

**Table 22:**

| Relationship between the weight fluctuation and fasting blood sugar level decreasing rate | | | | | |
|---|---|---|---|---|---|
| Body weight fluctuation | Reduction rate of fasting blood sugar level (%) | | | | Total |
| | >20% | 20-10% | <10% | No change or increase | |
| Reduction of 3 kg or more | 8 | 3 | 4 | 3 | 18 |
| Reduction of 3 kg or less | 6 | 8 | 6 | 4 | 24 |
| No change or increase | 3 | 8 | 7 | 11 | 29 |
| Kruskal-Wallis test, Hc=13.62>9.21, P<0.05 | | | | | |

**Table 23:**

| Relationship between the weight fluctuation and total serum cholesterol decreasing rate | | | | | |
|---|---|---|---|---|---|
| Body weight fluctuation | Reduction rate of fasting blood sugar level (%) | | | | Total |
| | >20% | 20-10% | <10% | No change or increase | |
| Reduction of 3 kg or more | 11 | 0 | 1 | 1 | 13 |
| Reduction of 3 kg or less | 18 | 2 | 1 | 4 | 25 |
| No change or increase | 9 | 6 | 5 | 11 | 31 |
| Kruskal-Wallis test, Hc=13.62>9.21, P<0.01 | | | | | |

The decreasing rate of total serum cholesterol is more strongly correlated to the reduction of body weight than that of fasting blood sugar, with a significance level of 1% or less. The body weight of the type II diabetes patients participated in this clinical test was larger by 24% than the standard body weight, suggesting that many obese persons were included. The strong relationship between reduction of body weight and total serum cholesterol clearly shows that it is necessary for a patient of multiple risk factor syndrome to take measures to avoid accumulation of viscera fat resulting from excessive intake of energy from food, and that burning the accumulated viscera fat is the first priority to improve the pathologic condition.

### Therapeutic effect on diabetic complications in Clinical test II

Diabetes is a complicating disease. Complications intrinsic to diabetes take place in the peripheral nerve, kidney, eye, skin, and so forth. The complications are developed when insulin sensitivity decreases. If the insulin tolerance is improved by medication, such complications should be improved. Then, results of 30 cases with diabetic complications were statistically analyzed and checked for therapeutic effect.

**Table 24:**

| Therapeutic effect of 4-0-methylascochlorin on diabetic complications | | | | | | | |
|---|---|---|---|---|---|---|---|
| Symptom | Presence or absence | Immediately before medication | 4th week | 8th week | 12th week | Total | Statistical significant difference |
| Throat dry | Present | 29 | 14 | 7 | 2 | 68 | P<0.005 |
| | Absent | 1 | 16 | 23 | 28 | 52 | |
| | Total | 30 | 30 | 30 | 30 | 120 | |
| Large amount of urine excretion | Present | 30 | 14 | 8 | 1 | 53 | P<0.005 |
| | Absent | 0 | 16 | 22 | 29 | 67 | |
| | Total | 30 | 30 | 30 | 30 | 120 | |
| Feeling of fatigue | Present | 14 | 10 | 2 | 0 | 26 | P<0.005 |
| | Absent | 1 | 5 | 13 | 15 | 34 | |
| | Total | 15 | 15 | 15 | 15 | 60 | |
| Feeling of weariness | Present | 25 | 15 | 6 | 1 | 47 | P<0.005 |
| | Absent | 0 | 10 | 19 | 24 | 53 | |
| | Total | 25 | 25 | 25 | 25 | 100 | |
| Pain in the calf of leg | Present | 12 | 7 | 6 | 2 | 27 | P<0.005 |
| | Absent | 4 | 9 | 10 | 14 | 37 | |
| | Total | 16 | 16 | 16 | 16 | 64 | |
| 1) test of significant difference by the Kruskal-Wallis test | | | | | | | |

As is apparently implied from the fact that the term "diabetes" is derived from "siphon" in Greek, a diabetic patient always feels thirsty and thus drinks a large amount of water, and consequently excretes a large amount of urine, like a siphon. Therefore "throat dry" and "excessive urination" are regarded as symptoms intrinsic to diabetic nephropathy. These symptoms have been significantly improved by medication. In other words, 4-0-methylascochlorin overcame insulin tolerance, thereby improving diabetic nephropathy. The "feeling of fatigue", "feeling of weariness", and "pain in the calf of leg" are also symptoms intrinsic to diabetic complications and collectively called diabetic neuropathy. As is shown in the table, 4-0-methylascochlorin also improved these symptoms with the passage of time. These results indicate that a compound of the present invention is also effective in improving diabetic neuropathy.

In Clinical test II, 5 cases of type II diabetic patients having 10-20 year's disease history were excluded. However, in these 5 cases, the effectiveness of the compound on complications was clearly observed. The effectiveness of a medicament is inversely proportional to the period of a disease. In other words, the longer the period of the disease, the lower the efficacy. In brief, the symptoms associated with diabetic nephropathy such as "throat dry" and "excessive urination" disappeared from all patients.

### Glucose tolerance improving effect in Clinical test II

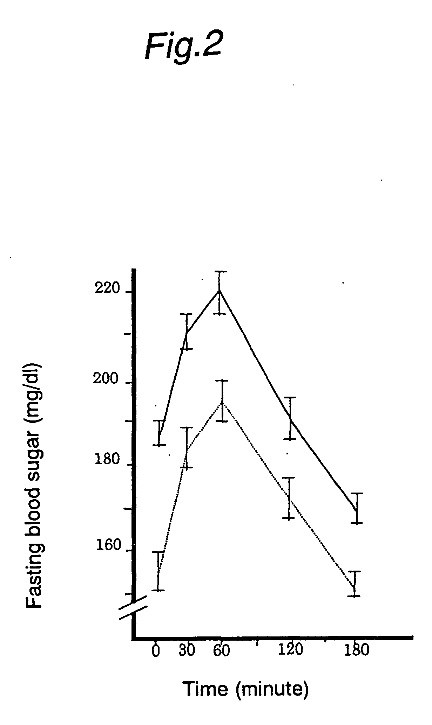

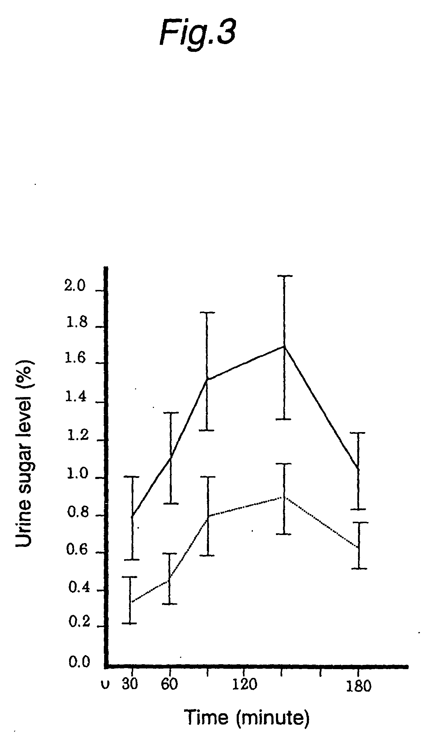

A glucose tolerance test were performed by perorally taking 50 g of glucose at fasting time in the early morning and measuring concentrations of blood sugar and urine sugar with time. The results are shown in Figures 2 and 3. A solid line represents the results immediately before therapy and a broken line represents immediately after the therapy of 12 weeks. Since a process of a glucose tolerance test more or less varied depending upon medical institutions, data of all patients (30 patients) with type II diabetes participated in this test in a single and the same institution are statistically analyzed and shown. Figure 2 shows blood sugar level in a glucose tolerance test and Figure 3 shows urine sugar level in the glucose tolerance test. Note that, in the Figures 2 and 3, numerical values each represent average value ± standard deviation.

As is apparent from theses figures, 4-0-methylascochlirin significantly decreases blood sugar level (measured) in a time dependent manner as compared to that before treatment. In addition, 4-0-methylascochlorin significantly decreases the concentration of glucose contained in the urine which is taken at the same time blood was taken. Since there results have a statistically high significant difference, it is clear that compounds of this series osvercomes insulin tolerance, thereby improving glucose tolerance.

Next, preparation examples will be described below.

### Preparation Example 1

### Dehydroascofuranone

Dehydroascofuranone, which is formed by removing two hydrogen atoms from a tetrahydrofuran ring of ascofuranone, was synthesized as follows: First, 0.16g (4.0 mml) of sodium hydroxide was dissolved in 0.7 ml of deionized water. While this mixture was cooled in a water bath, 0.7 ml of an aqueous deionized solution of AgNO₃ (0.34g, 2.00 mml) was added little by little to the mixture. To the water suspension solution of silver nitrate thus produced, 1.4 ml of a dioxane solution of ascofuranone (0.2g, 0.476 mmol) was added. The reaction mixture was stirred at room temperature for 3 hours, diluted with water-dioxane solvent mixture (1:1), and filtrated through cerite. The obtained filtrate was acidified with 6N-HCl and extracted with dichloromethane. The dichloromethane layer was washed with water and a saturated saline solution, dried over MgSO₄, concentrated under vacuum. The residue was subjected to column chromatography and purified with diochloromethane:acetone (60:1) to obtain dehydroascofuranone of 0.047 g (a yield of 24%). mp. 139.5-140.5°C IR (cm-1): 1680, 1635, 1555 ¹H NMR (ppm): 1.35 (6H, s), 1.82 (3H, br s), 1.84 (3H, br s), 2.13 (2H, br t, J=7.2 Hz), 2.33 (2H, br q, J=7.5 Hz), 2.60 (3H, s), 3.40 (2H, d, J=7.2 Hz). 10.14 (1H, s), 12.69 (1H, s) ¹³C NMR (ppm): 12.93, 14.44, 16.13, 22.03, 23.03 (probably two overlapping peaks), 27.05, 38.29, 88.10, 98.52, 113.14, 113.59, 114.21, 121.90, 125.76, 135.03, 137.71, 138.12, 156.17, 162.15, 184.66, 193.30, 207.20 Elemental analysis Found(%) C, 65.88; H, 6.54. Calculated(%) C, 65.94; H, 6.50

### Preparation Example 2

### A. 4-O-Ethoxycarbonylmethyl-2-O-methylascochlorin (1)

Sodium hydride (60%, 23.1 mg, 0.578 mmol) was washed with pentane and then suspended in DMF (1 ml). To the solution mixture, 2 ml of a DMF solution of 2-O-methylascochlorin (0.220 g, 0.525 mmol) was added dropwise and stirred at room temperature for 30 minutes. Thereafter, bromoethyl acetate (0.0641 ml, 0.578 mmol) was added to the mixture and stirred at 50°C for 4 hours. The reaction solution was poured in a saturated aqueous ammonia chloride solution and extracted with ether. The organic layer was washed with water and a saturated saline solution successively and dried over anhydrous magnesium sulfate. After the drying agent was filtered off, vacuum concentration was performed, and the residue was purified by silica gel column chromatography (SiO₂: 15 g, hexane/ethyl acetate) to obtain a substance (1) of 0.204 g (77%) as a colorless gum.
IR (film): 1765, 1713, 1694. NMR (CDCl₃, 500 MHz): 0.70 (3H, s), 0.80 (3H, d, J=6.7 Hz), 0.83 (3H, d, J=6.9 Hz), 1.31 (3H, t, J=7.0 Hz), 1.58-1.67 (1H, m), 1.90 (3H, br s), 1.90-1.96 (2H, m), 2.34-2.44 (3H, m), 2.63 (3H, s), 3.65 (2H, d, J=7.0 Hz), 3.88 (3H, s), 4.29 (2H, q, J=7.0 Hz), 4.61 (2H, s), 5.39 (1H, d, J=16.0 Hz), 5.46 (1H, t, J=7.0 Hz), 5.89 (1H, d, J=16.0 Hz), 10.42 (1H, s). ESMS (electrospray mass spectrum, positive) (m/z): 505 {(M+H)⁺, C₂₈H₃₇O₆³⁵Cl + H⁺}, 507 {(M+H)⁺, C₂₈H₃₇O₆³⁷Cl + H⁺}

### B. 4-O-Carboxymethyl-2-O-methylascochlorin (2)

The substance (1) (0.207 g, 0.409 mmol) obtained above was dissolved in methanol (6.2 ml) and 20% aqueous potassium carbonate solution (1ml, 1.45 mmol) was added thereto. The resultant mixture was stirred at room temperature for 2 hours. The reaction mixture was adjusted at pH2 with 3N hydrochloric acid and extracted with ether. The organic layer was washed with water and a saturated saline solution successively and dried over anhydrous magnesium sulfate. After the drying agent was filtered off and vacuum concentration was performed, the residue was purified by silica gel column chromatography (SiO₂: 15 g, dichloromethane/methanol) to obtain a substance (2) of 0.122 g (63%) as a colorless gum.
IR (film): 1715, 1696. NMR (CDCl₃, 500 MHz): 0.70 (3H, s), 0.81 (3H, d, J=6.8 Hz), 0.83 (3H, d, J=6.8 Hz), 1.62 (1H, qd, J=13.5, 6.0 Hz), 1.91 (3H, br s), 1.91-1.97 (2H, m), 2.34-2.45 (3H, m), 2.63 (3H, s), 3.62 (2H, d, J=6.9 Hz), 3.84 (3H, s), 4.65 (2H, s), 5.42 (1H, d, J=16.0 Hz), 5.43 (1H, t, J=6.9 Hz), 5.89 (1H, d, J=16.0 Hz), 10.42 (1H, s).

### Preparation Example 3

### 4-O-(4-Carboxybutanoyl)ascochlorin (4)

Ascochlorin (0.150 g, 0.358 mmol) was dissolved in anhydrous pyridine (1.5 ml). To the solution mixture, 4-dimethylaminopyridine (4.4 mg, 0.036 mmol) and glutaric anhydride (0.049 g, 0.430 mmol) were added. The reaction solution was stirred at 50°C overnight and then adjusted at pH2 by adding 1N hydrochloric acid and then subjected to extraction with ethyl acetate. The organic layer was washed with a saturated saline solution and dried over anhydrous magnesium sulfate. After the drying agent was filtered off, vacuum concentration was performed, and the residue was purified by silica gel column chromatography (SiO₂: 13 g, hexane/ethyl acetate) to obtain a substance (4) of 0.081 g (42%) as colorless crystal.
mp 120-123°C IR (KBr disc): -3000, 1771, 1711, 1649. NMR (CDCl₃, 500 MHz): 0.70 (3H, s), 0.81 (3H, d, J=7.0 Hz), 0.83 (3H, d, J=7.0 Hz), 1.59-1.66 (1H, m), 1.88 (3H, s), 1.89-1.96 (2H, m), 2.12 (2H, qui, J=7.2 Hz), 2.34-2.45 (3H, m), 2.55 (2H, t, J=7.2 Hz), 2.65 (3H, s), 2.76 (2H, t, J=7.2 Hz), 3.42 (2H, br d, J=6.0 Hz), 5.35 (1H, t, J=7.0 Hz), 5.39 (1H, d, J=16.0 Hz), 5.87 (1H, d, J=16.0 Hz), 10.30 (1H, s), 12.54 (1H, s).

### Preparation Example 4

### 4-O-isonicotinoyl-2-O-methylascochlorin (5)

2-O-methylascochlorin (61.3 mg, 0.146 mmol) was dissolved in anhydrous pyridine (1 ml). To the solution mixture, isonicotinoyl chloride hydrochloride (71.7 mg, 0.403 mmol) was added. After the reaction solution was stirred at room temperature for 3 hours, water was added thereto and further stirred for 30 minutes, and the solution was subjected to extraction with ethyl acetate. The organic layer was washed with a saturated aqueous cupper sulfate solution, water, a saturated aqueous sodium bicarbonate solution, and a saturated saline solution successively in this order, and thereafter dried over anhydrous magnesium sulfate. After the drying agent was filtered off, vacuum concentration was performed, and the residue was purified by silica gel column chromatography (SiO₂: 10 g, hexane/ethyl acetate) to obtain a substance (5) of 69.3 mg (90%) as a colorless gum.
IR (film): 1760, 1710, 1700. NMR (CDCl₃, 500 MHz): 0.69 (3H, s), 0.76-0.83 (6H, br), 1.59-1.64 (1H, m), 1.65 (3H, br s), 1.85-1.96 (2H, m), 2.32-2.45 (3H, m), 2.67 (3H, s), 3.39-3.50 (1H, br), 3.50-3.63 (1H, br), 3.88 (3H, s), 5.29 (1H, d, J=16.0 Hz), 5.34 (1H, t, J=7.0 Hz), 5.82 (1H, d, J=16.0 Hz), 7.79-7.99 (2H, m), 8.87 (2H, m), 10.49 (1H, s). ESMS (electrospray mass spectrum, positive) (m/z): 524 {(M+H)⁺, C₃₀H₃₄NO₅³⁵Cl + H⁺}, 526 {(M+H)⁺, C₃₀H₃₄NO₅³⁷Cl + H⁺}.

### Preparation Example 5

### 4-O-Isonicotinoylascochlorin (6)

Ascochlorin (0.200 g, 0.494 mmol) was dissolved in anhydrous pyridine (1 ml). To the solution mixture, isonicotinoyl chloride hydrochloride (0.242 g, 1.36 mmol) was added. After the reaction solution was stirred at room temperature for 3 hours, water was added thereto and further stirred for 30 minutes, and the solution was subjected to extraction with ether. The organic layer was washed with a saturated aqueous cupper sulfate solution, water, a saturated aqueous sodium bicarbonate solution, and a saturated saline solution, successively in this order, and thereafter dried over anhydrous magnesium sulfate. After the drying agent was filtered off, vacuum concentration was performed, and a crystallized product was purified by recrystallization from hexane/ether to obtain a substance (6) of 0.199 g (79%) as colorless crystal.
mp 119-121°C IR (film): 1752, 1711, 1642. NMR (CDCl₃, 500 MHz): 0.68 (3H, s), 0.79 (3H, d, J=7.0 Hz), 0.81 (3H, d, J=7.0 Hz), 1.59-1.65 (1H, m), 1.68 (3H, br s), 1.86-1.96 (2H, m), 2.33-2.44 (3H, m), 2.69 (3H, s), 3.34-3.46 (1H, br), 3.46-3.60 (1H, br), 5.29 (1H, d, J=16.0 Hz), 5.36 (1H, t, J=7.0 Hz), 5.82 (1H, d, J=16.0 Hz), 8.01 (2H, br d, J=4.6 Hz), 8.85-8.95 (2H, br), 10.35 (1H, s), 12.60 (1H, s). ESMS (electrospray mass spectrum, negative) (m/z): 508 {(M-H)⁻, C₂₉H₃₂NO₅³⁵Cl - H⁺}, 510 {(M-H)⁻, C₂₉H₃₂NO₅³⁷Cl - H⁺}.

### Preparation Example 6

### A. 4-O-Acetyl-2-O-metnylascofuranone (8); precursor of compound (1)

4-O-ascofuranone (0.301 g, 0.651 mmol) was dissolved in acetone (2 ml). The solution mixture, potassium carbonate (99 mg, 0.716 mmol) and methyl iodide (0.0484 ml, 0.781 mmol) were added and the reaction mixture was refluxed with heating for 2 hours. After the reaction solution was filtered, the filtrate was diluted with ether and washed with water, a saturated aqueous sodium bicarbonate solution, and a saturated saline solution, successively in this order, and thereafter dried over anhydrous magnesium sulfate. After the drying agent was filtered off, vacuum concentration was performed, the residue was purified by silica gel column chromatography (SiO₂: 10 g, hexane/ethyl acetate) to obtain a substance (8) of 75.5 mg (24%) as a colorless gum.
NMR (CDCl₃, 500 MHz): 1.22 (3H, s), 1.28 (3H, s), 1.64 (3H, s), 1.77 (3H, br s), 2.04 (2H, t, J=7.8 Hz), 2.09-2.20 (2H, m), 2.35 (3H, s), 2.39 (1H, dd, J=18.2, 10.0 Hz), 2.47 (1H, dd, J=18.0, 6.2 Hz), 2.63 (3H, s), 3.33 (2H, d, J=6.5 Hz), 3.83 (3H, s), 4.52 (1H, dd, J=10.0, 6.2 Hz), 5.08 (1H, tq, J=6.5, 1.3 Hz), 5.52 (1H, t, J=6.6 Hz), 10.44 (1H, s).

### B. 2-O-methylascofuranone (7)

The substance (8) obtained above (63.5 mg, 0.133 mmol) was dissolved in methanol (4.2 ml). To the dissolved solution, a 1% aqueous sodium hydroxide solution (4.2 ml, 1.05 mmol) was added. The solution mixture was stirred at room temperature for 4 hours. The reaction solution was neutralized with 2N hydrochloric acid and methanol was removed by distillation. The residue was diluted with water and extracted with ethyl acetate. The organic layer was washed with water, a saturated aqueous sodium bicarbonate solution, and a saturated saline solution successively in this order, and thereafter dried over anhydrous magnesium sulfate. After the drying agent was filtered off, vacuum concentration was performed, and the residue was purified by silica gel column chromatography (SiO₂: 10 g, hexane/ethyl acetate) to obtain a substance (7) of 52.1 mg (90%) as a colorless gum.
IR (film): 3390, 1756, 1686, 1564. NMR (CDCl₃, 500 MHz): 1.22 (3H, s), 1.29 (3H, s), 1.64 (3H, br s), 1.80 (3H, br s), 2.06 (2H, t, J=7.5 Hz), 2.12-2.22 (2H, m), 2.40 (1H, dd, J=18.0, 10.0 Hz), 2.47 (1H, dd, J=18.0, 6.5 Hz), 2.66 (3H, s), 3.42 (2H, d, J=6.6 Hz), 3.82 (3H, s), 4.53 (1H, dd, J=10.0, 6.5 Hz), 5.20 (1H, tq, J=6.9, 1.2 Hz), 5.51 (1H, t, J=6.9 Hz), 6.35 (1H, s), 10.37 (1H, s). ESMS (electrospray mass spectrum, negative) (m/z): 433 {(M-H)⁻, C₂₄H₃₁O₅³⁵Cl - H⁺}, 435 {(M-H)⁻, C₂₄H₃₁O₅³⁷Cl - H⁺}.

### Preparation Example 7

### 4-O-(4-Carboxybutanoyl)ascofuranone (9)

Sacofuranone (0.100 g, 0.238 mmol) was dissolved in anhydrous pyridine (1 ml). To the solution mixture, 4-dimethlaminopyridine (2.9 mg, 0.04 mmol) and glutaric anhydride (0.033 g, 0.490 mmol) were added. The reaction solution was stirred at 50°C overnight and adjusted at pH2 by adding 1N hydrochloric acid and then subjected to extraction with ethyl acetate. The organic layer was washed with a saturated saline solution and dried over anhydrous magnesium sulfate. After the drying agent was filtered off, vacuum concentration was performed, and the residue was purified by silica gel column chromatography (SiO₂: 10 g, hexane/ethyl acetate) to obtain a substance (9) of 0.040 g (32%) as a colorless gum.
IR (film): ∼3000, 1752, 1713, 1638. NMR (CDCl₃, 500 MHz): 1.23 (3H, s), 1.29 (3H, s), 1.63 (3H, br s), 1.74 (3H, br s), 2.02 (2H, t, J=7.5 Hz), 2.11 (2H, qui, J=7.0 Hz), 2.12-2.17 (2H, m), 2.37 (1H, dd, J=18.2, 10.0 Hz), 2.44 (1H, dd, J=18.2, 6.3 Hz), 2.54 (2H, t, J=7.0 Hz), 2.65 (3H, s), 2.76 (2H, t, J=7.0 Hz), 3.24-3.31 (2H, br), 4.55 (1H, dd, J=10.0, 6.3 Hz), 5.07 (1H, tq, J=7.0, 1.5 Hz), 5.50 (1H, br t, J=7.0 Hz), 10.30 (1H, s), 12.52 (1H, s).

### Preparation Example 8

### 4-O-Isonicotinoylascochlorin (10)

Ascofuranone (0.200 g, 0.476 mmol) was dissolved in anhydrous pyridine (1 ml). To the solution mixture, isonicotinoyl chloride hydrochloride (0.233 g, 1.31 mmol) was added. After the reaction solution was stirred at room temperature for one hour, water was added. The mixture was further stirred for 30 minutes and extracted with ether. The organic layer was washed with a saturated aqueous cupper sulfate solution, water, a saturated aqueous sodium bicarbonate solution, and a saturated saline solution successively in this order, and thereafter dried over anhydrous magnesium sulfate. After the drying agent was filtered off, vacuum concentration was performed, and the residue was purified by silica gel column chromatography (SiO₂: 10 g, hexane/ethyl acetate) to obtain a substance (10) of 0.135 g (54%) as a colorless gum.
IR (film): 1756, 1644. NMR (CDCl₃, 500 MHz): 1.21 (3H, s), 1.28 (3H, s), 1.53 (3H, br s), 1.62 (3H, br s), 1.88-1.97 (2H, m), 2.02-2.12 (2H, m), 2.35 (1H, dd, J=18.0, 10.0 Hz), 2.42 (1H, dd, J=18.0, 6.3 Hz), 2.69 (3H, s), 3.21-3.33 (1H, br), 3.33-3.46 (1H, br), 4.51 (1H, dd, J=10.0, 6.3 Hz), 5.09 (1H, br t, J=7.0 Hz), 5.48 (1H, br t, J=7.0 Hz), 8.00-8.02 (2H, m), 8.89-8.91 (2H, m), 10.35 (1H, s), 12.60 (1H, s). ESMS (electrospray mass spectrum, negative) (m/z): 524 {(M-H)⁻, C₂₉H₃₂NO₆³⁵Cl - H⁺}, 526 {(M-H)⁻, C₂₉H₃₂NO₆³⁷Cl - H⁺}

### Preparation Example 9

### Preparation of 2,4-Di-O-methylascochlorinic acid (11)

To 2,4-Di-O-methylascochlorin (0.428 g, 0.989 mmol), NaH₂PO₄•2H₂O (0.155 g, 0.0993 mmol) and water (2.6 ml) were added and the mixture was stirred to dissolve the phosphate. Subsequently, tert-butyl alcohol (10.4 ml) and 2-methyl-2-butene (0.460 ml, 4.34 mmol) were added and further 90% sodium chlorite (0.305 g, 3.04 mmol) was added to the mixture and stirred at room temperature for 15 minutes. The reaction solution was diluted with water and extracted with dichloromethane. The dichloromethane layer was washed with a saturated saline solution and dried over magnesium sulfate. The drying agent was filtered off and vacuum concentration was performed to obtain 0.396 g of a crude product, which was further purified with silica gel column chromatography (Merck, Silica gel 60, a particle size of 63 to 200 µm, 10g, developed with dichloromethane/ethanol) to obtain a substance (11) of 0.384 g (87%) as a colorless gum.
IR (KBr disk): 3440, 1712.
NMR (CD₃OD, 500 MHz): 0.70 (3H, s), 0.79 (3H, d, J=6.8 Hz), 0.82 (3H, d, J=6.8 Hz), 1.61 (1H, qd, J=13.4, 4.7 Hz), 1.92 (3H, s), 1.92-2.10 (2H, m), 2.24-2.29 (1H, m), 2.33 (3H, s), 2.50-2.62 (2H, m), 3.53 (2H, d, J=7.0 Hz), 3.80 (3H, s), 3.81 (3H, s), 5.46 (1H, t, J=7.0 Hz), 5.48 (1H, d, J=15.8 Hz), 5.93 (1H, d, J=15.8 Hz). ESMS (electrospray mass spectrum, negative) (m/z):447[(M-H)⁻, C₂₅H₃₃O₅³⁵Cl - H⁺], 449[(M-H)⁻, C₂₅H₃₃O₅³⁷Cl - H⁺],

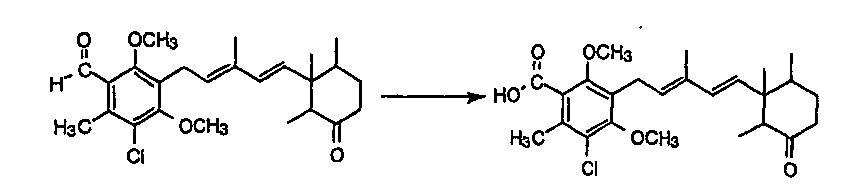

### Preparation Example 10

### Preparation of 2,4-Di-O-acetylascochlorin (12)

Ascochlorin (0.300 g, 0.741 mmol) was dissolved in anhydrous pyridine (1.3 ml). While the solution mixture was cooled in a water bath, acetyl chloride (0.158 ml, 2.22 mmol) was added dropwise to the solution mixture. After the reaction solution was stirred at room temperature for 4 hours, a saturated aqueous NaHCO₃ solution (2 ml) was added and further stirred for 20 minutes. The reaction solution was diluted with water and extracted with ether. The ether layer was washed with a saturated CuSO₄ solution, water and a saturated saline solution successively in this order and dried over anhydrous sodium sulfate. After the drying agent was filtered off, vacuum concentration was performed to obtain a substance (12) of 0.320 g (88%) as a colorless gum.
NMR (CDCl₃, 500 MHz): 0.71 (3H, s), 0.81 (3H, d, J=6.7 Hz), 0.84 (3H, d, J=6.7 Hz), 1.63 (1H, qd, J=13.0, 5.5 Hz), 1.86 (3H, s), 1.90-1.97 (2H, m), 2.34 (3H, s), 2.35 (3H, s), 2.36-2.43 (3H, m), 3.35 (2H, d, J=7.0 Hz) 5.25 (1H, t, J=7.0 Hz), 5.41 (1H, d, J=16.0 Hz), 5.87 (1H, d, J=16.0 Hz), 10.27 (1H, s).

### Preparation Example 11

### Preparation of 2,4-Di-O-acrtylascochlorinic acid (13)

To 2,4-Di-O-acetylascochlorin (0.320 g, 0.650 mmol), NaH₂PO•2H₂O (0.103 g, 0.660 mmol) and water (1.8 ml) were added. The solution mixture was stirred to dissolve the phosphate. Subsequently, tert-butyl alcohol (7.2 ml) and 2-methyl-2-butene (0.309 ml, 2.92 mmol) were added and further 90% sodium chlorite (0.200 g, 1.99 mmol) was added to the mixture and stirred at room temperature for 15 minutes. The reaction solution was diluted with water and extracted with dichloromethane. The dichloromethane layer was washed with a saturated saline solution and dried over anhydrous sodium sulfate. The drying agent was filtered off and vacuum concentration was performed to obtain 0.290 g of a crude product, which was further purified with silica gel column chromatography (Merck, Silica gel 60, a particle size of 63 to 200 µm, 10g, eluted with dichloromethane/isopropyl alcohol) to obtain a substance (13) of 0.211 g (64%) as a colorless gum.
IR (KBr disc): 3450, 1781, 1735, 1712.
NMR (CD₃OD, 500 MHz): 0.71 (3H, s), 0.81 (3H, d, J=6.8 Hz), 0.83 (3H, d, J=6.8 Hz), 1.62 (1H, qd, J=13.0, 4.8 Hz), 1.86 (3H, s), 1.93-1.99 (1H, m), 1.99-2.06 (1H, m), 2.23 (3H, s), 2.27 (1H, ddd, J=13.2, 4.8, 2.0 Hz), 2.28 (3H, s), 2.42 (3H, s), 2.51-2.58 (2H, m), 3.33 (2H, d, J=6.9 Hz), 5.25 (1H, t, J=6.9 Hz), 5.50 (1H, d, J=16.0 Hz), 5.89 (1H, d, J=16.0 Hz).
ESMS (electrospray mass spectrum, negative) (m/z): 503[(M-H)⁻, C₂₇H₃₃O₇³⁵Cl-H⁺], 505[(M-H)⁻, C₂₇H₃₃O₇³⁷Cl-H⁺].

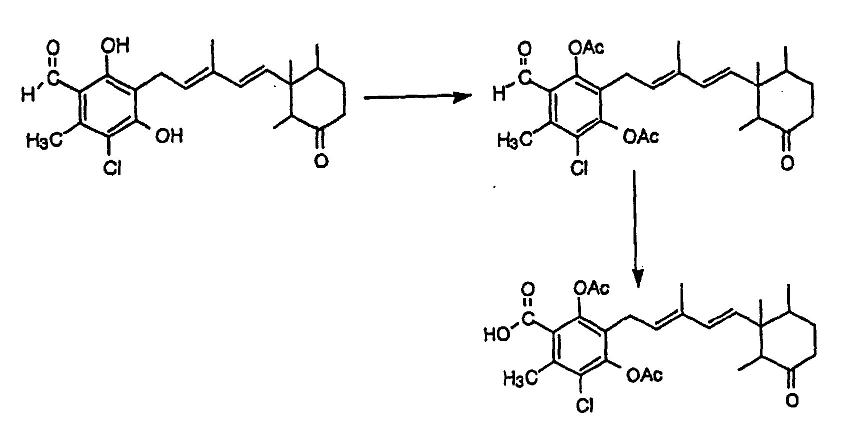

### Preparation Example 12

### Preparation of 2,4-Di-O-t-butyldimethylsilylascochlorin (14)

Ascochlorin (0.500 g, 1.23 mmol) was dissolved in anhydrous dimethylformamide (2.5 ml). To this mixture, imidazole (0.421 g, 6.18 mmol) and t-butyldimethylsilylchloride (0.559 g, 3.71 mmol) were added. The reaction solution was stirred at room temperature for 2.5 hours and a saturated aqueous NaHCO₃ solution (1.25 ml) was added thereto and further stirred for 20 minutes. After the resultant reaction solution was diluted with water, it was extracted with ether. The ether layer was washed with water, a saturated aqueous NaHCO₃ solution, a saturated saline solution, successively in this order, and dried over anhydrous potassium carbonate. After the drying agent was filtered off, vacuum concentration was performed to obtain a crude product of 0.9364 g, which was further purified by silica gel column chromatography (eluted with hexane/ethyl acetate: 20:1). As a result, a substance (14) of 0.707 g (90%) was obtained as a colorless gum.
NMR (CDCl₃, 500 MHz): 0.13 (6H, s), 0.26 (6H, s), 0.70 (3H, s), 0.81 (3H, d, J=6.7 Hz), 0.83 (3H, d, J=6.7 Hz), 1.02 (9H, s), 1.03 (9H, s), 1.62 (1H, qd, J=13.5, 5.0 Hz), 1.79 (3H, s), 1.89-1.96 (2H, m), 2.32-2.45 (3H, m), 2.63 (3H, s), 3.45 (2H, d, J=5.9 Hz), 5.33 (1H, d, J=16.0 Hz), 5.41 (1H, t, J=5.9 Hz), 5.87 (1H, d, J=16.0 Hz), 10.29 (1H, s).

### Preparation Example 13

### Preparation of ascochlorinic acid (15)

To 2,4-Di-O-t-butyldimethylsilyascochlorin (14) (0.450 g, 0.710 mmol), NaH₂PO₄•2H₂O (0.112 g, 0.811 mmol) and water (3 ml) were added. The solution mixture was stirred to dissolve the phosphate. Subsequently, to this mixture, tert-butyl alcohol (12 ml) and 2-methyl-2-butene (0.333 ml, 3.96 mmol) were added and 90% sodium chlorite (0.219 g, 2.18 mmol) was further added and stirred at room temperature for 45 minutes. The reaction solution was diluted with a saturated saline solution and extracted with dichloromethane. The dichloromethane layer was washed with a saturated saline solution and dried over anhydrous sodium sulfate. After the drying agent was filtered off, vacuum concentration was performed to obtain an oily product 2, which was further dissolved in anhydrous tetrahydrofuran (7 ml). To this solution, 1M hydrofluoric acid (2.00 ml, 2.00 mmol) and 1M aqueous sodium fluoride solution (6.40 ml, 6.40 mmol) were added. After the reaction solution was stirred at room temperature for 2 days, it was diluted with water and extracted with dichloromethane. The extracted solution was dewatered and dried by adding anhydrous magnesium sulfate. The drying agent was filtered off and vacuum concentration was performed to obtain a crude product of 0.414 g. The crude product was purified by silica gel column chromatography (extracted with dichloromethane/methanol: 10:1) to obtain a substance (5) of 0.276 g (92%) as a colorless gum. To this substance, ether was added and crystallized to obtain a substance (15) of 0.163 g (55%) as a colorless microcrystalline solid.
m.p. 156-158°C
IR (KBr disc): 3430, 1705, 1599, 1564.
NMR (CD₃OD, 500 MHz): 0.69 (3H, s), 0.80 (3H, d, J=6.7 Hz), 0.82 (3H, d, J=6.7 Hz), 1.60 (1H, qd, J=13.3, 4.8 Hz), 1.91 (3H, s), 1.91-2.03 (2H, m), 2.26 (1H, ddd, J=13.3, 4.8, 1.8 Hz), 2.50-2.58 (2H, m), 2.66 (3H, s), 3.49 (2H, d, J=7.3 Hz), 5.40 (1H, d, J=16.0 Hz), 5.53 (1H, t, J=7.3 Hz), 5.91 (1H, d, J=16.0 Hz).
ESMS (electrospray mass spectrum, negative) (m/z): 419[(M-H)⁻, C₂₃H₂₉O₅³⁵Cl - H⁺], 421[(M-H)⁻, C₂₃H₂₉O₅³⁷Cl - H].

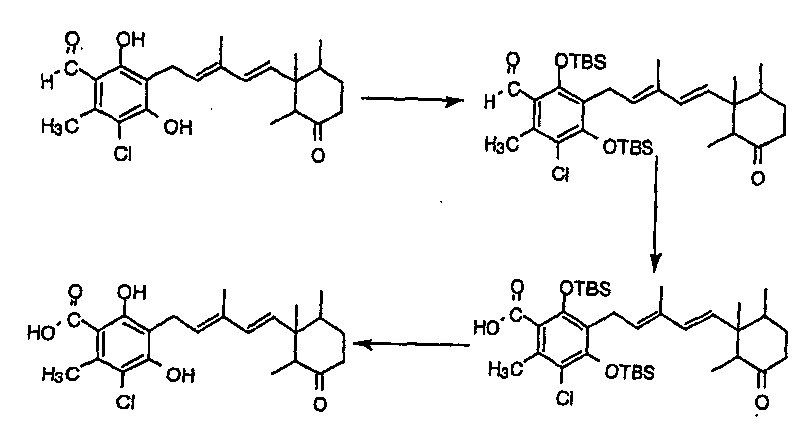

### INDUSTRIAL APPLICABILITY

The aforementioned ascochlorin, ascochlorin, ascofuranone or an ascofuranone homologue of the present invention in which hydrogen of the hydroxyl group at the 2-and/or 4-position of the olsyl aldehyde is substituted by a specific group, is useful in diagnosing, preventing and treating multiple risk factor syndrome.

## Claims

1. A preventive or therapeutic agent for multiple risk factor syndrome, comprising, as an active ingredient, one or more chemical compounds selected from the group consisting of ascochlorin, ascochlorin homologues, ascofuranone and ascofuranone homologues,
each of the chemical compounds having at least an olsyl aldehyde moiety, in which
neither of the hydrogen atoms of the 2- and 4-hydroxyl groups are substituted; and
the hydrogen atom of at least one of the hydroxyl groups is substituted by a group selected from the group consisting of (C₁-C₁₅) alkyl, (C₂-C₁₅) alkenyl, aryl, (C₁-C₁₅) alkylaryl, -(CₙH₂ₙ)R (where n is an integer of 1 to 15 and R represents COOR' which substitutes for any one of n carbon atoms where R' is H or (C₁-C₁₅) alkyl), (C₁-C₁₅) alkylcarbonyl, arylcarbonyl, aryl(C₁-C₁₅)alkyl, aryl (C₁-C₁₅) alkylcarbonyl, -C(=O)(CH₂)₁₋₁₅COOH, 2-pyridylcarbonyl, nicotinoyl, isonicotinoyl, toluenesulfonyl, carbamoyl, carbamoyl substituted by one or two (C₁-C₆) alkyls, amino, amino substituted by one or two (C₁-C₆) alkyls, aryloxy (C₁-C₁₅) alkyl optionally having a halogen on the ring, and aryl having a (C₁-C₆) alkoxy or a (C₁-C₆) alkoxycarbonyl on the ring.

2. The preventive or therapeutic agent for multiple risk factor syndrome according to claim 1, wherein the compound is represented by the formula: 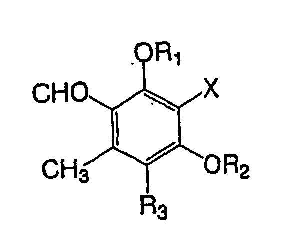 where X is represented by 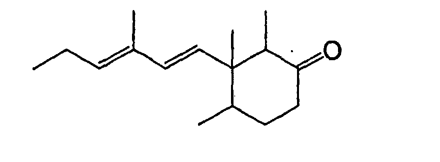 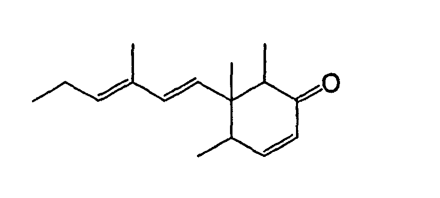 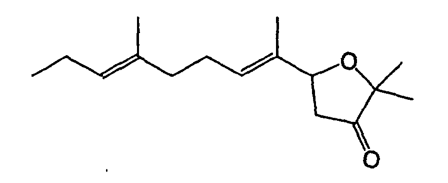 , or 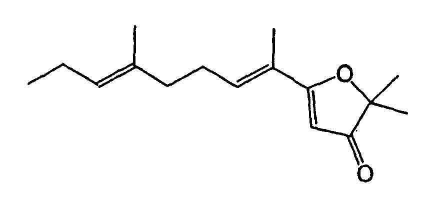 ;
R₁ is H, (C₁-C₁₅) alkyl, (C₂-C₁₅) alkenyl, or (C₁-C₁₅) alkycarbonyl;
R₂ is H, (C₁-C₁₅) alkyl, (C₂-C₁₅) alkenyl, aryl, (C₁-C₁₅) alkylaryl, (CₙH₂ₙ)R (where n is an integer of 1 to 15 and R represents COOR' which substitutes for any one of n carbon atoms where R' is H or (C₁-C₁₅) alkyl), (C₁-C₁₅) alkylcarbonyl, arylcarbonyl, aryl (C₁-C₁₅)alkyl, aryl (C₁-C₁₅) alkylcarbonyl, -C(=O)(CH₂)₁₋₁₅COOH, 2-pyridylcarbonyl, nicotinoyl, isonicotinoyl, toluenesulfonyl, carbamoyl, carbamoyl substituted by one or two (C₁-C₆) alkyls, amino, amino substituted by one or two (C₁-C₆) alkyls, aryloxy (C₁-C₁₅) alkyl optionally having a halogen on the ring, and aryl having a (C₁-C₆) alkoxy or a (C₁-C₆) alkoxy on the ring; and
R₃ is H or a halogen,
with the proviso that a case where X is represented by the formula: 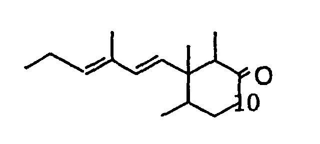 ,
R₁ is H, R₂ is H, and R₃ is Cl is excluded.

3. The preventive or therapeutic agent for multiple risk factor syndrome according to claim 2 wherein the compound is represented by the formula: 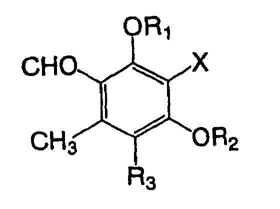 where X is represented by 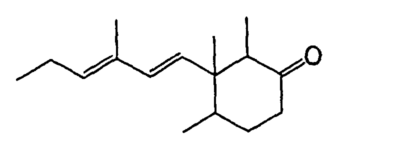 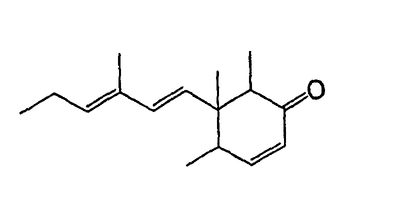 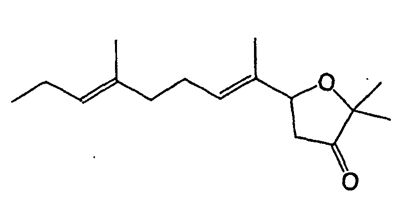 or 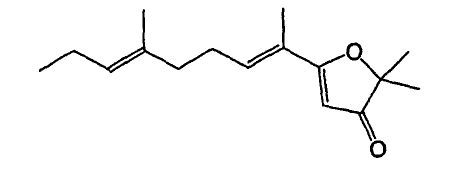 ;
R₁ is H, (C₁-C₆) alkyl, (C₂-C₆) alkenyl, or (C₁-C₆) alkycarbonyl;
R₂ is H, (C₁-C₆) alkyl, (C₂-C₆) alkenyl, aryl, (C₁-C₆) alkylaryl, -(CₙH₂ₙ)R (where n is an integer of 1 to 6 and R represents COOR' which substitutes for any one of n carbon atoms where R' is H or (C₁-C₆) alkyl), (C₁-C₆) alkylcarbonyl, arylcarbonyl, aryl(C₁-C₆)alkyl, aryl(C₁-C₆) alkylcarbonyl, -C(=O)(CH₂)₁₋₆COOH, 2-pyridylcarbonyl, nicotinoyl, isonicotinoyl, toluenesulfonyl, carbamoyl, carbamoyl substituted by one or two (C₁-C₃) alkyls, amino, amino substituted by one or two (C₁-C₃) alkyls, aryloxy (C₁-C₆) alkyl optionally having a halogen on the ring, and aryl having a (C₁-C₃) alkoxy or a (C₁-C₃) alkoxy on the ring; and
R₃ is H or Cl,
with the proviso that a case where X is represented by the formula: 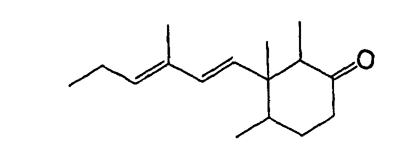 ,
R₁ is H, R₂ is H, and R₃ is Cl, is excluded.

4. A preventive or therapeutic method for multiple risk factor syndrome comprising administering one or more chemical compounds according to any one of claims 1 to 3 to a patient requiring therapy.

5. A kit for a preventive or therapeutic agent for multiple risk factor syndrome, comprising one or more chemical compounds according to any one of claims 1 to 3 and an instruction for use.

6. Use of a compound according to any one of claims 1 to 3 for manufacturing a preventive or therapeutic agent for multiple risk factor syndrome.

7. A compound, represented by the following formula, stereoisomer, optical isomer, or a pharmaceutically acceptable salt thereof 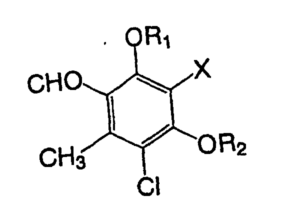 ,
where X is 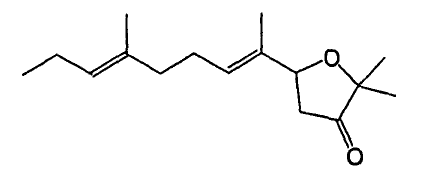 R₁ is H, (C₁-C₆) alkyl, (C₂-C₆) alkenyl, or (C₁-C₆) alkycarbonyl;
R₂ is H, -CH₂COOH, -CH₂COO(C₁-C₅)alkyl, (C₁-C₆) alkycarbonyl, -C(=O)(CH₂)₁₋₆COOH, 2-pyridylcarbonyl, nicotinoyl, or isonicotinoyl; and
R₃ is H or Cl,
with the proviso that a case where R₁ is H, R₂ is H, and R₃ is Cl, is excluded; or
X is represented by the formula: 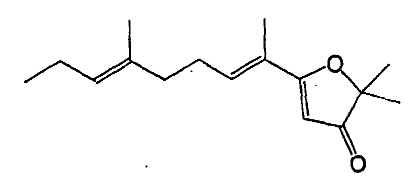 R₁ is H, (C₁-C₆) alkyl, (C₂-C₆) alkenyl, or (C₁-C₆) alkycarbonyl;
R₂ is H, (C₁-C₆) alkyl, (C₂-C₆) alkenyl, -CH₂COOH, -CH₂COO(C₁-C₆)alkyl, (C₁-C₆) alkycarbonyl, -C(=O)(CH₂)₁₋₆COOH, 2-pyridylcarbonyl, nicotinoyl, or isonicotinoyl; and
R₃ is H or Cl.

8. The compound represented by the following formula, a stereoisomer, optical isomer, or a pharmaceutically acceptable salt thereof: 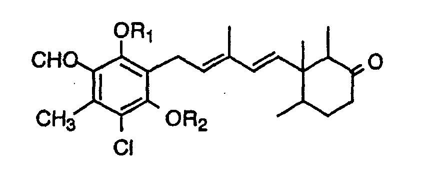 where R₁ is H, -(C₁-C₅) alkyl or alkenyl, or -C(=O)(C₁-C₅) alkyl ; and
R₂ is H, -(C₁-C₅) alkyl, -CH₂COOH, -CH₂COO(C₁-C₅)alkyl, -C(=O) (C₁-C₅)alkyl, -C(=O) (CH₂)₁₋₅COOH, nicotinoyl, or isonicotinoyl,
with the proviso that a case where R₁ is H, R₂ is -CH₂COOH or nicotinoyl is excluded.

9. A pharmaceutical composition comprising one or more chemical compounds according to claim 7 or 8 and a pharmaceutically acceptable carrier.

10. A preventive or therapeutic agent for multiple risk factor syndrome, comprising one or more chemical compounds according to claim 7 or 8, as an active ingredient.
